# EUROPEAN PATENT APPLICATION

(11) **EP 2 468 260 A2**
(43) Date of publication of application: **27.06.2012**
(21) Application number: 10809583.7
(22) Date of filing: 12.08.2010
(51) Int. Cl.: A61K 9/133, A61K 47/48, A61P 35/00, A61P 37/00

(54) **LIPOSOMES COVERED WITH THE EXTRACELLULAR DOMAIN OF THE APO2L/TRAIL PROTEIN**

(30) Priority: 21.08.2009 ES 200930618
(71) Applicant: Universidad De Zaragoza, 50009 Zaragoza (ES); Universidad Del Pais Vasco-Euskal Herriko Unibertsitatea, 48940 Leioa (Vizcaya) (ES); Consejo Superior De Investigaciones Científicas, 28006 Madrid (ES)
(72) Inventor: ANEL BERNAL, Luis Alberto, E-50009 Zaragoza (ES); MARTÍNEZ LORENZO, María José, E-50009 Zaragoza (ES); MARTÍNEZ LOSTAO, Luis, E-50009 Zaragoza (ES); ÁLAVA MARTÍNEZ DE CONTRASTA, María Ángeles, E-50009 Zaragoza (ES); LARRAD MUR, Luis, E-50009 Zaragoza (ES); NAVAL IRABERRI, Javier, E-50009 Zaragoza (ES); PIÑEIRO ANTÕN, Andrés, E-50009 Zaragoza (ES); BASAÑEZ ASUA, Gorka, E-48940 Leioa (Vizcaya) (ES)
(74) Representative: Pons Ariño, Angel
(86) International application number: PCT/ES2010/000354
(87) International publication number: WO 2011/020933

(57) **Abstract**

The invention relates to the field of biotechnology and medicine. The invention specifically relates to a compound comprising a liposome covered or decorated at least with the extracellular domain of the APO2L/TRAIL protein, and to the use thereof for developing a medicament preferably for the treatment of a cancer or inflammatory or autoimmune diseases, such as rheumatoid arthritis.

## Description

The present invention belongs to the field of biotechnology and medicine. Specifically, the present invention relates to a compound that comprises a liposome covered or decorated, at least, with the extracellular domain of the APO2L / TRAIL protein, and to the use thereof for the preparation of a medicament, preferably for the treatment of a cancer or of inflammatory or autoimmune diseases, such as rheumatoid arthritis.

### PRIOR STATE OF THE ART

APO2L / TRAIL is a protein of the TNF (Tumour Necrosis Factor) family which acts as a deadly ligand, inducing cell death by activating the extrinsic apoptotic pathway (Pitti et al. 1996. J Biol Chem 271: 12687-90; Wiley et al. 1995. Immunity 3: 673-82). APO2L / TRAIL is capable of inducing the apoptosis of tumour cells without affecting normal cells. This observation has caused the signalling pathway of this molecule to have a great therapeutic potential in the treatment of cancer (Mitsiades et al. 2001. Blood 98: 795-804; Marini et al. 2006. Oncogene 25: 5145-54; Mitsiades et al. 2001. Expert Opin Investig Drugs 10: 1521-30; Duiker et al. 2006. Eur J Cancer 42: 2233-40; Daniel et al. 2007. Blood 110: 4037-46; Xu et al. 2003. World J Gastroenterol 9: 1241-5). The use of various recombinant forms of APO2L / TRAIL in pre-clinical trials to induce the apoptosis of tumour cells, either in monotherapy, or combined with other therapies, has been disclosed in the patent applications with publication numbers EP1957529, US6444640, CA2544923, W09701633, W002069995 and W003028001. In fact, the use thereof for the treatment of various malignant neoplasms is currently being evaluated in various clinical trials (Johnstone et al. Nat Rev Cancer. 2008 Oct; 8: 782-98).

APO2L / TRAIL has also been involved in the process called Activation-Induced Cell Death (AICD), whereby the activated T lymphocytes that are generated during an immune response are eliminated in order to recover homeostasis (Anel et al. 2007. Front Biosci 12: 2074-84; Bosque et al. 2008. J Leukoc Biol 84: 488-98; Bosque et al. 2005. J Leukoc Biol 77: 568-78; Bosque A et al. 2005 Eur J Immunol 35: 1812-21; Martinez-Lorenzo et al. 1998. Eur J Immunol 28: 2714-25). The involvement of APO2L / TRAIL in immune system regulation phenomena and in the pathogenesis of autoimmune diseases has been corroborated in the studies performed for APO2L / TRAIL on the KO mouse (Lamhamedi-Cherradi et al. 2003. Nat Immunol 4: 255-60). Rheumatoid Arthritis (RA) is an autoimmune-based disease, wherein the immune system attacks and basically destroys the joints. It is believed that a defective apoptosis could contribute to the perpetuation of the activated T lymphocytes present in the synovial fluid, which are responsible for the articular destruction characteristic of RA. For example, although the activated T lymphocytes present in the synovial fluid of patients with RA are sensitive to the APO2L /TRAIL-induced cell death, the synovial fluid of these patients seems to have a defect in the secretion of APO2L / TRAIL associated with exosomes (Martinez-Lorenzo et al. 2007. Clin Immunol 122: 28-40). The use of recombinant APO2L / TRAIL may also be an effective therapy for the treatment of RA (Yao et al. 2006. Arthritis Res Ther 8: R16; patent application with publication number WO0122987).

On the other hand, liposomes are lipid vesicles that are used as systems for the storage or transport of drugs designed for the treatment of various diseases, such as, for example, cancer or RA. The binding of ligands to the surface of liposomes which favour the vectoring of liposomes towards target cells is also well-known (Samad et al. 2007. Curr Drug Deliv 4: 297-305; Schwendener. 2007. Adv Exp Med Biol 620: 117-28; Metselaar et al. 2004. Ann Rheum Dis 63: 348-53; Ceponis et al. 2001. Arthritis Rheum 44: 1908-16; Trif et al. 2007. J Liposome Res 17: 237-48).

Therefore, the use of recombinant APO2L / TRAIL is a useful therapeutic approach for the treatment of cancer, as well as of inflammatory or autoimmune diseases, such as, for example, RA. However, the development of compounds that enhance its therapeutic activity and / or reduce the necessary therapeutic dose would make it possible to significantly improve the efficacy of the treatment and / or the patients' quality of life.

### EXPLANATION OF THE INVENTION

The present invention relates to a compound that comprises a liposome covered or decorated, at least, with the extracellular domain of the APO2L /TRAIL protein and to the use thereof for the preparation of a medicament, preferably for the treatment of a cancer or of inflammatory or autoimmune diseases, such as rheumatoid arthritis.

The authors of the present invention have generated artificial liposomes with characteristics similar to those of the natural exosomes secreted by cells as regards the lipid composition and the size thereof, whereto the bioactive APO2L / TRAIL protein has been incorporated on the surface (LUV-APO2L /TRAIL).

In cytotoxicity studies performed *in vitro* on different tumour cell lines, it has been demonstrated that the capacity to induce cell death is greater with LUV-APO2L / TRAIL than with soluble APO2L / TRAIL. Specifically, the mean inhibitory concentration is about 5 times lower in the case of liposomes decorated with APO2L / TRAIL as compared to the soluble protein, as the mean of the tumour cell lines analysed.

On the other hand, using an animal model of antigen-induced arthritis (AIA), it has been demonstrated that the intra-articular injection of LUV-APO2L /TRAIL is capable of reducing the inflammation of joints affected by arthritis more effectively that when the soluble APO2L / TRAIL protein is injected.

APO2L / TRAIL is a type II transmembrane protein that belongs to the TNF superfamily. The APO2L / TRAIL protein of *Homo sapiens* has 281 amino acids and its GenBank accession number is NP003801.1 (SEQ ID: 3). It has an intracellular domain (amino acids 1-17), a transmembrane domain (amino acids 18-38) and an extracellular domain (amino acids 39-281; SEQ ID: 2). This extracellular domain may be released into the medium and, thus, may appear in soluble form. Several recombinant soluble APO2 / TRAIL proteins with different sizes have been generated, which contain, for example, amino acids 91-281, 95-281 or 114-281 (SEQ ID: 1). These recombinant proteins have been used to study the relationship between the structure and the function of APO2L / TRAIL. Moreover, these recombinant soluble APO2L / TRAIL proteins are being commercialised by different commercial firms, such as, for example, Calbiochem®, with catalogue number 616376, Bender MedSystems®, with catalogue number BMS356, Prepotech®, with catalogue number 310-4, Biovisión, Inc., with catalogue number 4354-50, Cell Sciences®, with catalogue number CRT500, or Chemicon® GF092.

A first aspect of the present invention, which hereinafter will be called "composition of the invention", relates to a compound that comprises a liposome covered or "decorated", at least, with the protein with amino acid sequence SEQ ID: 2 or any of the biologically active variants or portions thereof.

In the sense used in this description, the term "variant" refers to a protein that is substantially homologous and functionally equivalent to the protein with amino acid sequence SEQ ID: 2. In general, a variant includes additions, deletions or substitutions of amino acids which do not substantially alter the function thereof. The term "variant" also includes the proteins resulting from post-translational modifications, such as, for example, without being limited thereto, glycosylation or phosphorylation.

As used herein, a protein is "substantially homologous" to the protein with amino acid sequence SEQ ID: 2 when its amino acid sequence presents a good alignment with the amino acid sequence of SEQ ID: 2, i.e. when its amino acid sequence has a degree of identity with respect to the amino acid sequence of SEQ ID: 2 of, at least, 60 %, typically of, at least, 80 %, advantageously of, at least, 85 %, preferably of, at least, 90 %, more preferably of, at least, 95 %, and even more preferably of, at least, 99 %. The sequences homologous to the sequence of SEQ ID: 2 may be easily identified by a person skilled in the art with the aid of an appropriate sequence comparison computer programme.

The terms "biologically active" and "functionally equivalent", as used in the present description, refer to any variant of the protein with amino acid sequence SEQ ID: 2, any portion of the protein with amino acid sequence SEQ ID: 2 or any portion of a variant of the protein with amino acid sequence SEQ ID: 2, which, either in soluble form, or as a part of the composition of the invention, maintains its function in relation to the capacity to induce apoptosis. Some apoptosis analysis methods are, for example, without being limited thereto, the morphological analysis of the condensation and / or fragmentation of chromatin and the apoptotic bodies, DNA fragmentation detection assays, analysis of apoptosis-induced proteases (PARP or caspase 3), methods based on DNA staining (DAPI or propidium iodide), analysis of membrane alterations (Annexin V), analysis of the cellular capacity to reduce MTT, *in situ* labelling techniques (TUNEL or ISEL), and detection of proteins related to apoptosis (Fas, Apo-1, CD59 or p53). For example, the capacity to induce apoptosis may be determined by means of conventional methods, such as the assays described in the Examples that accompany this description.

The term "portion" refers to a fragment of the protein with amino acid sequence SEQ ID: 2 or a fragment of a variant thereof. In a preferred embodiment, the present invention relates to a compound that comprises a liposome covered or "decorated", at least, with a biologically active portion of the protein which presents an identity with SEQ ID NO: 1 of, at least, 80 %, preferably of, at least, 90 %, more preferably of, at least, 95 %, and even more preferably of 100 %. The sequences homologous to the sequence of SEQ ID: 1 may be easily identified by a person skilled in the art with the aid of an appropriate sequence comparison computer programme.

Therefore, the terms "liposome decorated or covered with the extracellular domain of the APO2L / TRAIL protein", "liposome decorated or covered with the APO2L / TRAIL protein" and "liposome decorated or covered with APO2L / TRAIL", as used in the present description, refer to a liposome which has the protein of amino acid sequence SEQ ID: 2 or any of the biologically active variants or portions thereof bound to its surface. Preferably, said terms refer to a biologically active portion of the protein that presents an identity with SEQ ID NO: 1 of, at least, 80 %, more preferably of, at least, 90 %, even more preferably of, at least, 95 %, and even more preferably, of 100 %.

Liposomes are hollow vesicles that encapsulate part of the solvent wherein they have been prepared and the membrane whereof is formed by one or several lipid bilayers. They have been used as drug storage or transport systems for the treatment of various diseases, such as, for example, cancer or RA. However, the present invention discloses the therapeutic use of liposomes with a molecule anchored on the exterior thereof in order to enhance the bioactivity (in this case, increase the cytotoxic capacity of the APO2L / TRAIL protein, which acts as a deadly ligand, inducing the apoptosis of target cells). Thus, the liposome decorated with the APO2L / TRAIL protein becomes the treatment *per se* and not a mere drug reservoir or transport mode.

Liposomes may be classified as a function of their structure and their size (Vanee and Vanee, 1996). Large multilaminar liposomes (MLVs) are composed of more than one bilayer, which are arranged in a concentric form and separated from each other by wide aqueous spaces. On the contrary, unilaminar liposomes are composed of a single bilayer; they may likewise be classified according to their size into: small unilaminar liposomes (SUVs), when they have a mean diameter of between 25 and 40 nm, and large unilaminar liposomes (LUVs), when they have a mean diameter of between 40 and 500 nm. Preferably, the present invention relates to a compound that comprises an LUV with a diameter of between 100 nm and 200 nm.

Liposomes are predominantly formed from amphiphilic compounds, i.e. compounds **characterised in that** they have both a hydrophilic group (soluble in water) and a hydrophobic group (insoluble in water) in the same molecule. The most important structural components of liposomes are phospholipids. The properties of liposomal vesicles will depend, amongst other factors, on the nature of the constitutive phospholipids, certain characteristics such as the charge of their polar group and / or the length and the degree of saturation of their fatty acid chains. Some examples of phospholipids used are: phosphatidylcholines, phosphatidylethanolamines, phosphatidylserines, phosphatidylglycerols, phosphatidylinositols, phosphatidic acids and sphingomyelins. Preferably, the present invention relates to a compound that comprises a liposome composed of, at least, one phospholipid selected from: phosphatidylcholine and / or sphingomyelin.

In addition to phospholipids, other lipids may be a part of the composition of the liposomes. The liposomes may contain sterols, such as, for example, without being limited thereto, cholesterol or biliary acids; sphingolipids, such as, for example, without being limited thereto, gangliosides or cerebrosides; or chelating lipids. Preferably, the present invention relates to a compound that comprises a liposome which comprises, at least, one phospholipid selected from: phosphatidylcholine and / or sphingomyelin, and which further comprises a sterol. Preferably, the sterol is cholesterol.

The liposomes may be prepared by means of numerous processes known in the state of the art, such as, for example, without being limited thereto, sonication, extrusion, use of freezing-thawing cycles or use of surfactants.

The present invention relates to a compound that comprises a liposome covered with the APO2L / TRAIL protein. The soluble portion of the APO2L /TRAIL protein may be directly bound to the lipids that make up the liposome or through another molecule that is integrated within the liposome. This binding may be covalent, or by means of the formation of a coordinate compound with a chelated metal. The metal may be chelated in such a way that the coordinate bond takes place between a chelating group present in the liposome and a chelating group present in the protein. Said chelating groups may be metal chelating parts, such as a metal chelating lipid or a part used as a "metal affinity tag".

The terms "chelating part" and "metal chelating group" include any part that is capable of forming a coordination complex with an ionic metal. The term "metal chelating lipid", as used in the present description, refers to a lipid that comprises a metal chelating part or group, such as, for example, without being limited thereto, nitrile triacetic acid (NTA). In principle, any metal may be incorporated which has free coordination sites that may bind to electron donour elements of the metal chelating group. The metallic ion may be a transition metal ion, a lanthanide ion or an actinide ion. The metal may be selected from the group that comprises, for example, without being limited thereto, copper, nickel, zinc, iron, cobalt, cadmium, manganese and magnesium. Preferably, the metal is nickel.

The expression "metal affinity tag", as used in the present description, refers to a part that is capable of binding to a metallic ion by means of chelation, and includes, without being limited thereto, short amino acid sequences bound to the APO2L / TRAIL protein. The "tag" may be adjacent to the amino-terminal end of the APO2L / TRAIL protein. For example, without being limited thereto, a common "metal affinity tag" is made up of several histidine residues. Increasing the number of residues capable of binding to the metal generally increases the binding affinity. The APO2L / TRAIL protein with a "metal affinity tag" may be obtained by means of different recombinant protein generation techniques known in the state of the art, and the resulting protein may be isolated and purified by means of different methods known in the state of the art. As a consequence of the process of generating the recombinant protein, the latter may present an insertion of one or more extra amino acids between the affinity tag and the soluble portion of the APO2L / TRAIL protein.

Preferably, the present invention relates to a compound that comprises a liposome covered, at least, with the APO2L / TRAIL protein, which, bound to the N-terminal end thereof, has, at least, 6 histidines and, preferably, at least 10 histidines, and where the liposome further comprises a chelating lipid. Preferably, the chelating lipid is 1,2-dioleoyl-sn-glycero-3-[(N-(5-amino-1-carboxypentyl)iminodiacetic acid)succinyl] functionalised with nickel (DOGS-NTA-Ni).

Therefore, the compound of the invention preferably contains phosphatidylcholine, sphingomyelin, cholesterol and DOGS-NTA-Ni, where the proportion of DOGS with respect to the total weight of lipids is, at least, 1 %, more preferably, at least, 2.5 % and, even more preferably, at least 5 %. Therefore, some valid compositions are, for example, without being limited thereto, 59 % / 30 % / 10 % / 1 %, 57.5 % / 30 % /10 % / 2.5 % or 55 % / 30 % /10 % / 5 % of phosphatidylcholine, sphingomyelin, cholesterol and DOGS-NTA-Ni, respectively.

Another aspect of the invention relates to the use of the compound of the invention for the preparation of a medicament.

Another aspect of the invention relates to the use of the compound of the invention for the preparation of a medicament designed for the prevention or treatment of a cancer. The terms "tumour" and "tumoral", as used in the present description, refer to transformed cells which present an uncontrolled growth. Depending on the possible evolution thereof, it may be a benign tumour, which remains at its initiation site and does not produce metastasis, or a malignant tumour or cancer, which is invasive or produces metastasis. Therefore, the terms "cancer" and "cancerous", as used in the present description, refer to an alteration of the tumour cells which have the capacity to invade tissues or produce metastasis at sites distant from the primary tumour. Examples of cancer may be, without being limited thereto, breast, ovary, uterus, cervix, vagina, vulva, colon, bladder, prostate, skin, lung, liver, esophagus, stomach, pancreas, urinary tract, thyroid, kidney and brain cancer, sarcoma, plasmacytoma, myeloma, lymphoma and leukemia. Preferably, the present invention relates to the use of the compound of the invention for the preparation of a medicament designed for the prevention or treatment of a cancer belonging to the following group: lung, colon and kidney cancer, plasmacytoma, myeloma, lymphoma and leukemia. More preferably, the present invention relates to the use of the compound of the invention for the preparation of a medicament designed for the prevention or treatment of a cancer belonging to the following group: lung cancer, plasmacytoma, myeloma, lymphoma and leukemia.

Another aspect of the present invention relates to the use of the compound of the invention for the preparation of a medicament designed for the prevention or treatment of inflammatory or autoimmune diseases. The term "inflammatory diseases", as used in the present description, refers to those diseases wherein lesions are produced caused by an immune reaction or an inflammatory reaction of the body. Some examples of inflammatory diseases are, for example, without being limited thereto, conjunctivitis, iritis, uveitis, retinitis, otitis, mastoiditis, rhinitis, laberynthitis, sinusitis, pharyngitis, tonsillitis, bronchitis, pneumonia, bronchopneumonia, pleuritis, mediastinitis, endocarditis, thrombophlebitis, polyarteritis, nephritis, cystitis, stomatitis, esophagitis, gastritis, colitis, appendicitis, hepatitis, cholecystitis, pancreatitis, peritonitis, thyroiditis, dermatitis, encephalitis, meningitis, opitical neuromyelitis, polyneuritis, polymyositis, ossifying myositis, osteoarthritis and rheumatoid arthritis. Some inflammatory diseases, such as rheumatoid arthritis, are autoimmune disorders. The concept "autoimmune diseases" includes different pathological processes that evolve with an immune response against the body itself. Examples of autoimmune diseases include, without being limited thereto, Crohn's disease, pernicious anaemia, type I diabetes, Addison's disease, celiac disease, Graves' disease, multiple sclerosis, systemic lupus erythematosus, myasthenia gravis, Reiter's syndrome, Sjogren's syndrome, Hashimoto's thyroiditis, scleroderma, Goodpasture's syndrome, Wegener's granulomatosis, polymyalgia rheumatica and rheumatoid arthritis. Preferably, the present invention relates to the use of the compound of the invention for the preparation of a medicament designed for the prevention or treatment of rheumatoid arthritis.

Another aspect of the invention relates to a pharmaceutical composition that comprises the compound of the invention. In a preferred embodiment of this aspect of the invention, the pharmaceutical composition further comprises a pharmaceutically acceptable vehicle.

Another aspect of the invention relates to a pharmaceutical composition that comprises the compound of the invention and, moreover, jointly with a pharmaceutically acceptable vehicle, another active principle.

As used herein, the terms "active principle", "active substance", "pharmaceutically active substance", "active ingredient" and "pharmaceutically active ingredient" mean any component that potentially provides a pharmacological activity or a different effect in the diagnosis, cure, mitigation, treatment or prevention of a disease, or which affects the structure or function of the body of humans or other animals. The terms include those components that promote a chemical change during the preparation of the drug and which are present therein in a predicted modified form that provides the specific activity or effect.

The compositions of the present invention may be formulated for administration to an animal, more preferably a mammal, including a human being, in a variety of forms known in the state of the art. Thus, they may be, without being limited thereto, in aqueous or non-aqueous solutions, emulsions or suspensions. Examples of non-aqueous solutions are, for example, without being limited thereto, propylene glycol, polyethylene glycol, vegetable oils, such as olive oil, and injectable organic esters, such as ethyl oleate. Examples of aqueous solutions are, for example, without being limited thereto, water, water-alcohol solutions and saline media. The aqueous solutions may or may not be buffered, and may have additional active or inactive components. The additional components include salts to modulate the ionic strength, preservatives, including, without being limited thereto, anti-microbial agents, antioxidants, chelating agents or similar, and nutrients, including glucose, dextrose, vitamins and minerals. Alternatively, the compositions may be prepared for administration in solid form. The compositions may be combined with several inert vehicles or excipients, including, without being limited thereto: binding agents, such as microcrystalline cellulose, tragacanth or gelatin; excipients, such as starch or lactose; dispersing agents, such as alginic acid or corn starch; lubricants, such as magnesium stearate, sliding agents, such as colloidal silicon dioxide; sweetening agents, such as sucrose or saccharin; and flavouring agents, such as mint or methylsalicylate.

Such compositions and / or the formulations thereof may be administered to an animal, including a mammal and, therefore, a human being, in a variety of forms, including, without being limited thereto, intraperitoneal, intravenous, intramuscular, subcutaneous, intrathecal, intraventricular, intra-articular, intratumoral, oral, enteral, parenteral, intranasal, ocular or topical. A preferred administration route for the compositions and / or formulations of the compound of the invention designed for the prevention or treatment of a cancer is the intratumoral route. A preferred administration route for the compositions and / or formulations of the compound of the invention designed for the prevention or treatment of rheumatoid arthritis is the intra-articular route.

The dosage to obtain a therapeutically effective quantity depends on a variety of factors, such as, for example, the age, weight, sex or tolerance of the mammal. In the sense used in this description, the expression "therapeutically effective quantity" refers to the quantity of the compound or the pharmaceutical composition of the invention that produces the desired effect and, in general, will be determined, amongst other factors, by the characteristics of said compound or said pharmaceutical composition and the therapeutic effect to be achieved. The "adjuvants" and "pharmaceutically acceptable vehicles" that may be used in said compositions are the vehicles known in the state of the art.

Throughout the description and the claims, the word "comprises" and the variants thereof are not intended to exclude other technical characteristics, additives, components or steps. For persons skilled in the art, other objects, advantages and characteristics of the invention will arise partly from the description and partly from the practise of the invention. The following figures and examples are provided for illustrative purposes, and are not intended to limit the scope of the present invention.

### DESCRIPTION OF THE FIGURES

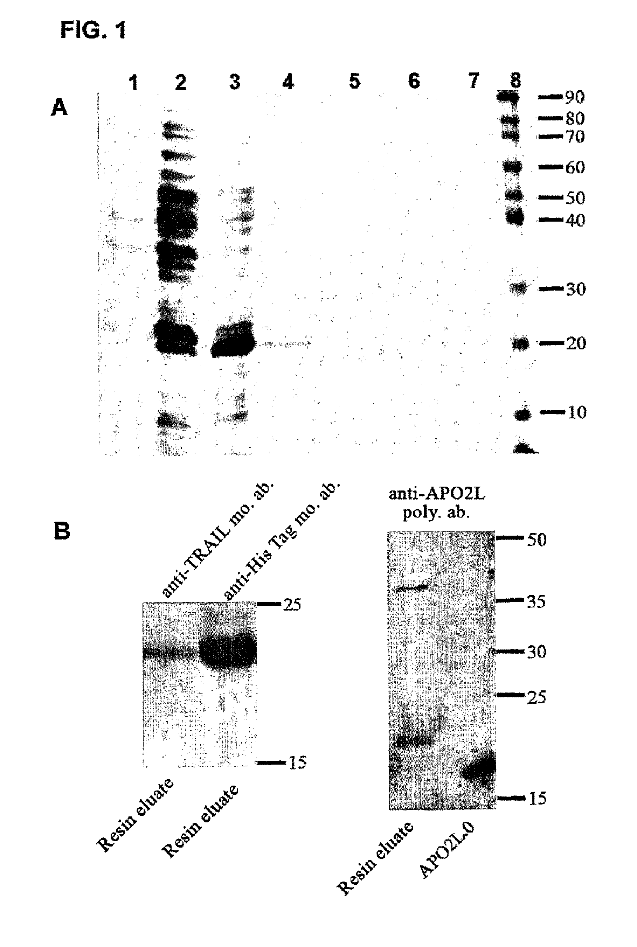

Figure 1 shows the expression and purification of recombinant APO2L /TRAIL. A. 12 % SDS-PAGE and Coomassie blue staining of the different expression and purification steps of APO2L / TRAIL-His₁₀: 1- E. Coli BL21-CodonPlus® (DE3)-RP transformed without IPTG (isopropyl-beta-D-thiogalactoside); 2- E. Coli BL21-CodonPlus® (DE3)-RP transformed and treated for 18 hours with 1 mM IPTG; 3- Precipitate of the diluted and dialysed bacterial extract; 4- Diluted and dialysed bacterial extract; 5- Supernatant of the extract after incubation with the resin; 6- Washing of the resin; 7- Resin eluate; 8- Molecular weight markers. B. Western-blot of the elution fraction of the resin developed with the Purified Mouse Anti-Human TRAIL monoclonal antibody, the His-Tag® Monoclonal Antibody, which recognises the histidine tail included in expression vector pET19b, and with a rabbit polyclonal antiserum previously generated in our laboratory, which recognises APO2L / TRAIL. As a control, the recombinant APO2L.0 protein without a histidine tail (Genentech, San Diego, CA) was also developed.

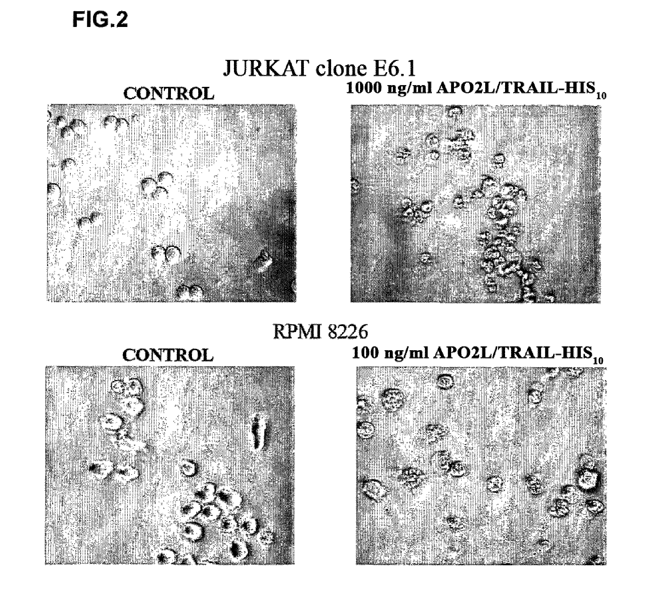

Figure 2 shows the cytotoxicity analysis of recombinant APO2L / TRAIL. The Jurkat clone E6.1 cell line and the RPMI 8226 cell line are incubated in the absence (Control) or in the presence of 1,000 ng / ml or 100 ng / ml of APO2L /TRAIL-His₁₀, respectively. After 24 hours, the presence of numerous cells with a typically apoptotic morphology is observed in both tumour cell lines.

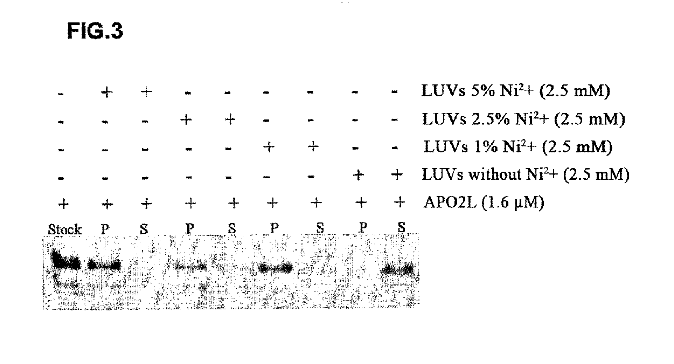

Figure 3 shows the binding of APO2L / TRAIL-His₁₀ to the LUVs with DOGS-NTA-Ni. LUVs were generated with a proportion of DOGS-NTA-Ni of 5, 2.5, 1 and 0 % (w / w), and were incubated with the APO2L / TRAIL-His₁₀ protein. Subsequently, the LUVs-APO2L / TRAIL are collected by means of ultracentrifugation and 12 % SDS-PAGE is performed by taking aliquots of both the collected pellets and the supernatants. The gel is stained with Coomassie blue. 1- APO2L / TRAIL-His₁₀ not incubated with LUVs; 2 and 3- Pellet and supernatant of LUVs-APO2L / TRAIL with 5 % DOGS-NTA-Ni; 4 and 5- Pellet and supernatant of LUVs-APO2L / TRAIL with 2.5 % DOGS-NTA-Ni; 6 and 7-Pellet and supernatant of LUVs-APO2L / TRAIL with 1 % DOGS-NTA-Ni; 8 and 9- Pellet and supernatant of LUV-APO2L / TRAIL with 0 % DOGS-NTA-Ni. Stock: APO2L / TRAIL-His₁₀; P: Pellet; S: Supernatant.

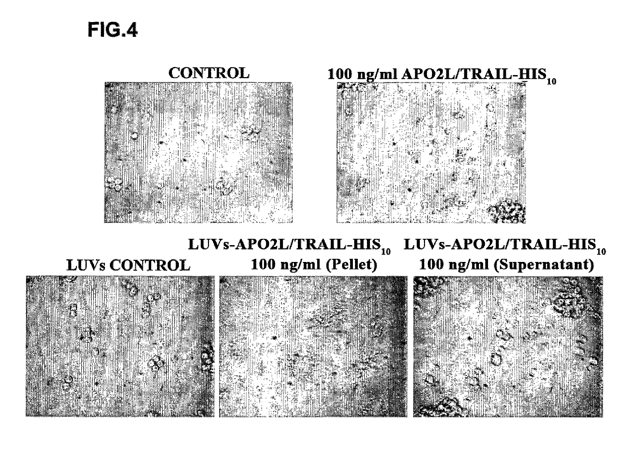

Figure 4 shows the cytotoxicity analysis of the LUVs-APO2L / TRAIL. The Jurkat clone E6-1 cell line is incubated in the presence of 100 ng / ml of APO2L / TRAIL-His₁₀, 100 ng / ml of LUVs 5 % Ni-APO2L / TRAIL present in the pellet obtained after the ultracentrifugation, 100 ng / ml of the supernatant or control 5 % LUVs that were not incubated with APO2L / TRAIL-His₁₀. After 24 hours, the presence of numerous cells with a typically apoptotic morphology is observed when the cells have been incubated with APO2L / TRAIL-His₁₀ and with LUVs 5 % Ni-APO2L / TRAIL present in the pellet. In the case of the supernatant or the control LUVs, no apoptosis of the Jurkat cells is observed.

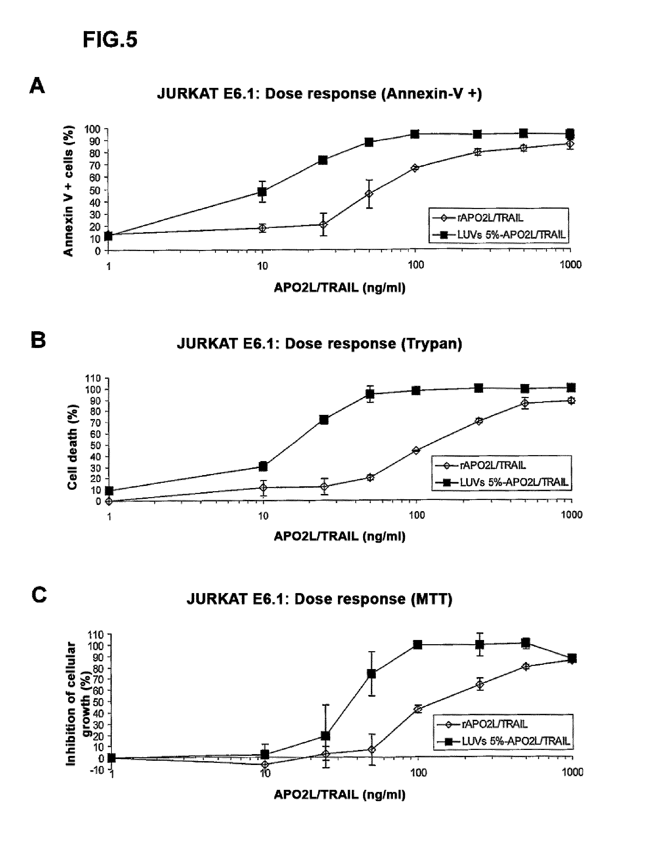

Figure 5 shows the *in vitro* cytotoxicity analysis of LUVs-APO2L / TRAIL in Jurkat clone E6.1. The capacity of both soluble APO2L / TRAIL and LUVs-APO2L / TRAIL to induce cell death was analysed in the Jurkat clone E6.1 tumour cell line. The cytotoxicity assay was performed by incubating the cells with doses of 1,000, 500, 250, 100, 50, 25 and 10 ng / ml for 24 hours. A. Analysis by flow cytometry to evaluate the presence of positive Annexin-V cells. B. Analysis by means of Trypan blue staining. C. Analysis with a variant of Mossman's MTT (dimethyl-thiazolyl-tetrazolium) reduction method. The results obtained are shown as the mean ± the standard deviation of at least two experiments.

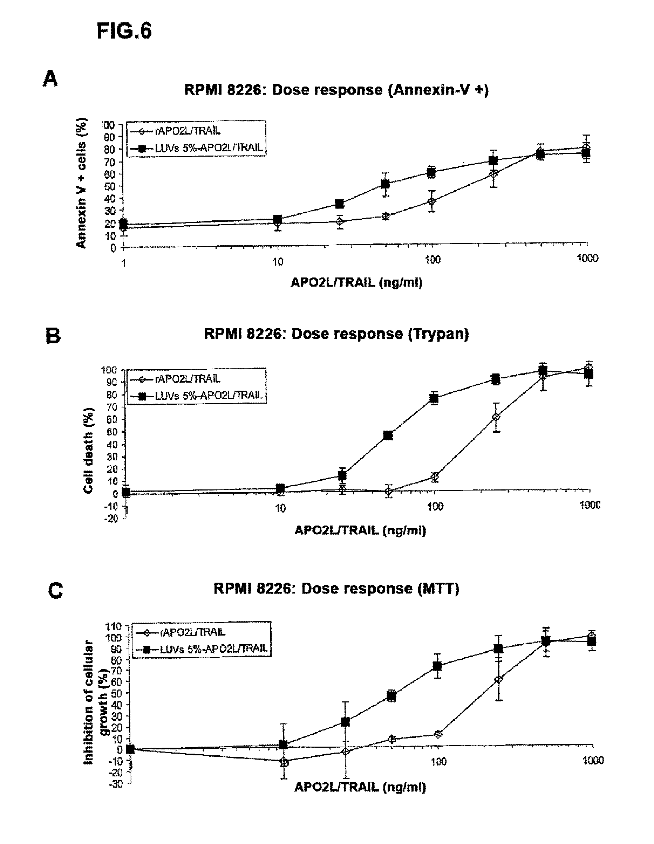

Figure 6 shows the *in vitro* cytotoxicity analysis of the LUVs-APO2L /TRAIL in RPMI 8226. The capacity of both soluble APO2L / TRAIL and the LUVs-APO2L / TRAIL to induce cell death in the RPMI 8226 tumour cell line was analysed. The cytotoxicity assay was performed by incubating the cells with doses of 1,000, 500, 250, 100, 50, 25 and 10 ng / ml for 24 hours. A. Analysis by flow cytometry to evaluate the presence of positive Annexin-V cells. B. Analysis by means of Trypan blue staining. C. Analysis with a variant of Mossman's MTT reduction method. The results obtained are shown as the mean ± the standard deviation of at least two experiments.

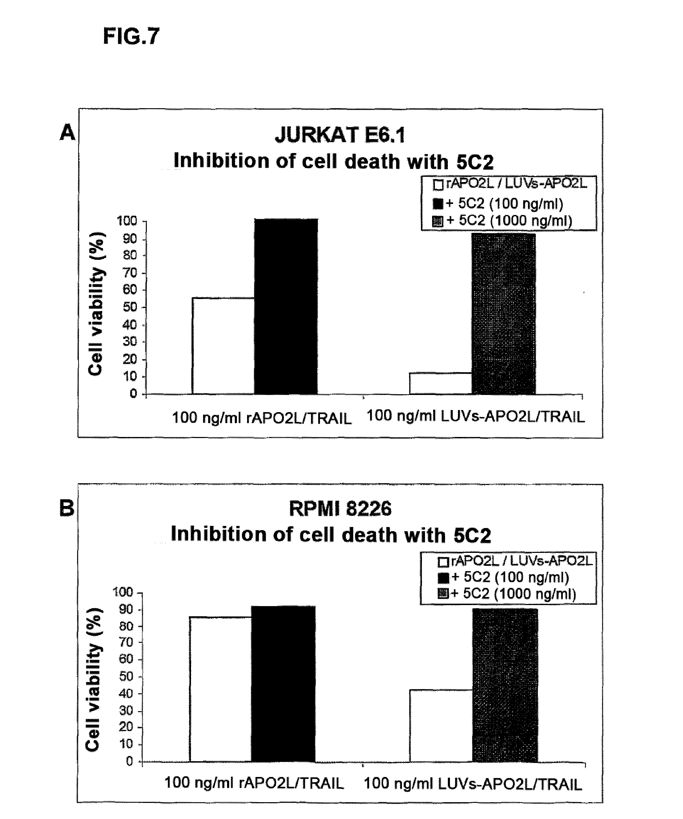

Figure 7 shows the *in vitro* cytotoxicity inhibition analysis with the blocking anti-APO2L / TRAIL 5C2 antibody. A cytotoxicity assay was performed in the absence or in the presence of the blocking anti-APO2L / TRAIL 5C2 antibody. A 100-ng / ml dose of 5C2 was used when it was incubated in the presence of soluble APO2L / TRAIL and a 1,000-ng / ml dose was used when it was incubated with LUVs-APO2L / TRAIL. A. Jurkat clone E6.1. B. RPMI 8226.

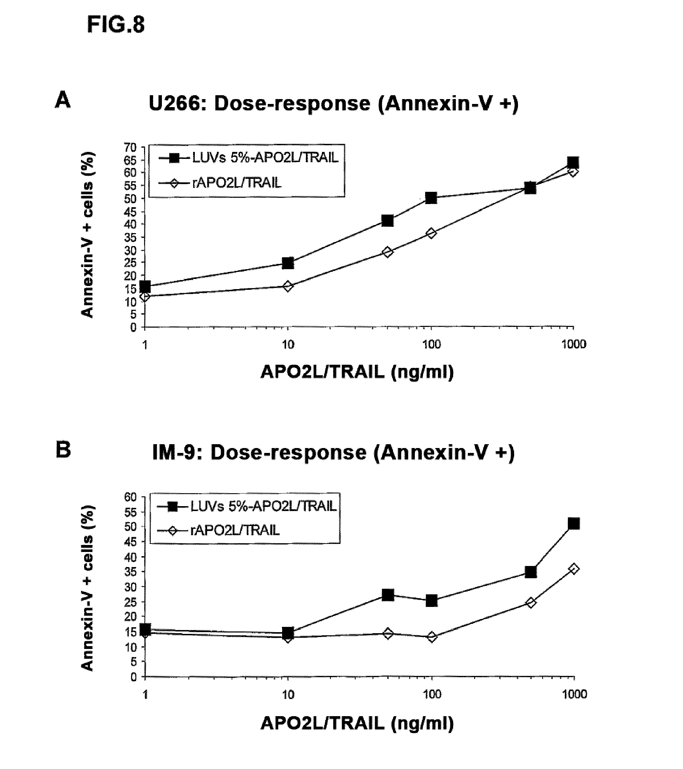

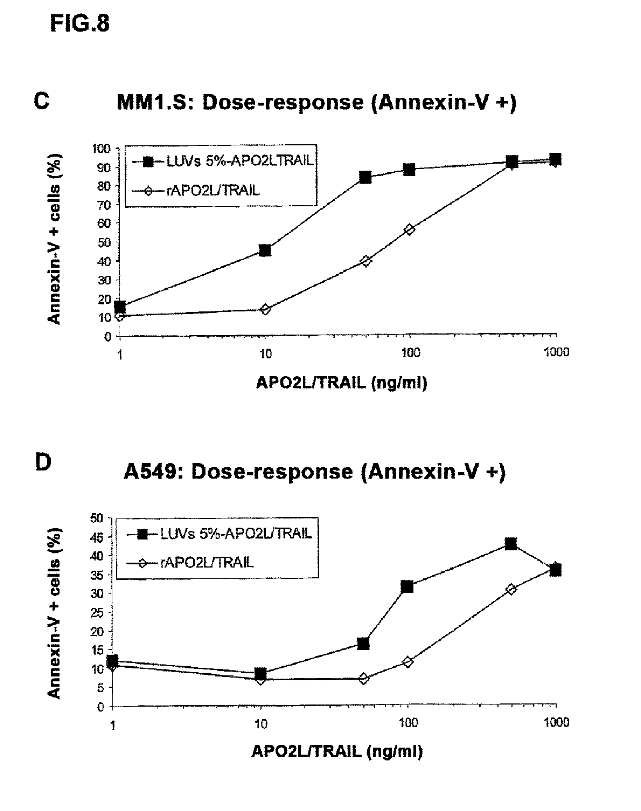

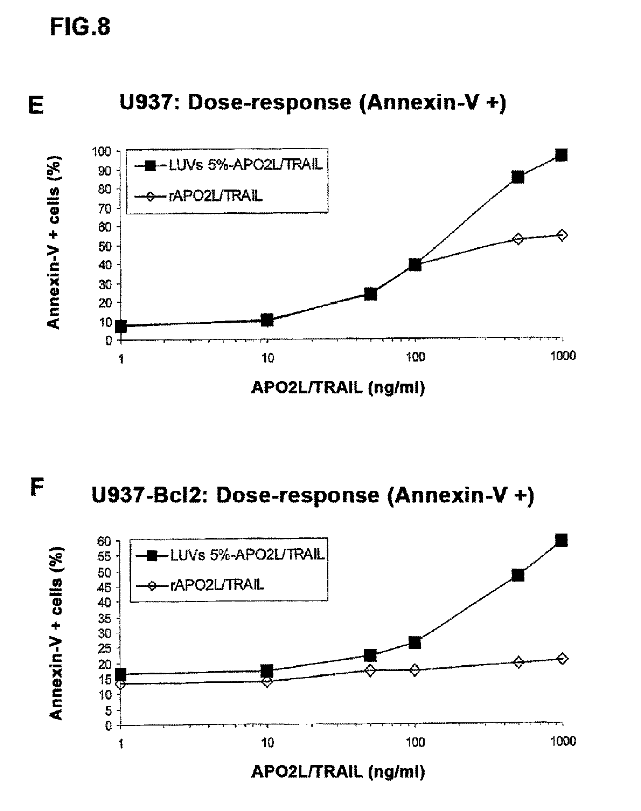

Figure 8 shows the *in vitro* cytotoxicity analysis of the LUVs-APO2L /TRAIL in U266, IM-9, MM1.S, A549, U937 and U937-Bcl2 (from the U937 parental line, wherein the anti-apoptotic Bcl2 protein has been overexpressed). The capacity of both soluble APO2L / TRAIL and the LUVs-APO2L / TRAIL to induce cell death was analysed by flow cytometry (positive Annexin-V cells). The cytotoxicity assay was performed by incubating the cells with doses of 1,000, 500, 100, 50 and 10 ng / ml for 24 hours. A. U266 cell line. B. IM-9 cell line. C. MM1.S cell line. D. A549 cell line. E. U937 cell line. F. U937-Bcl2 cell line.

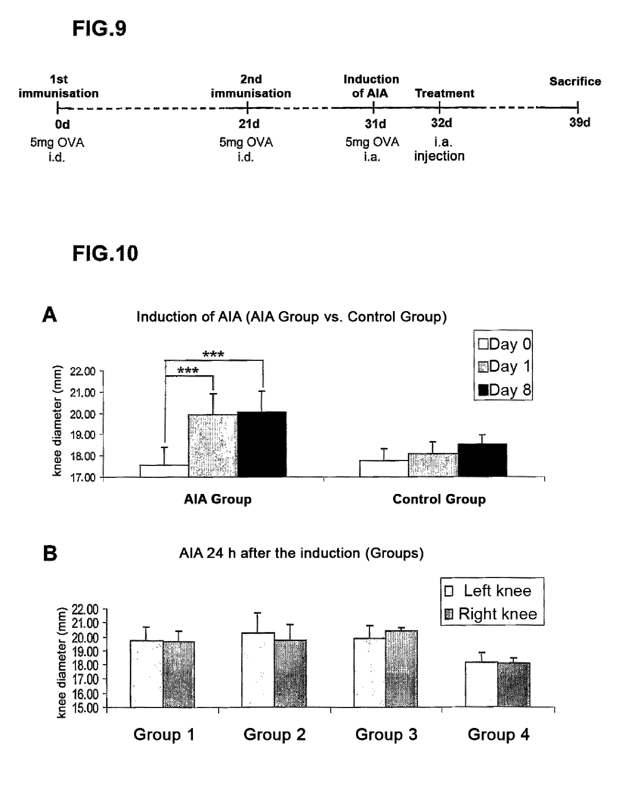

Figure 9 shows the time diagram of the protocol used for the induction of AIA.
Figure 10 shows the induction of antigen-induced arthritis (AIA) in New Zealand white rabbits. A. The i.a. injection with 5 mg of ovalbumin (OVA) correctly induces AIA in all the knees of the 13 New Zealand white rabbits (AIA Group). After 24 hours, there is a significant increase in the latero-lateral diameter of the knees, which is maintained until the day of the sacrifice. In the 6 rabbits wherein the i.a. injection is made with PBS (Control Group), there is no significant increase in the latero-lateral diameter of the knees. *** p < 0.001. B. AIA is produced in a similar manner in both knees and in the 3 treatment groups. The results obtained from the measurement of the latero-lateral diameter of the knees are shown as the mean ± the standard deviation of the animals in each group.

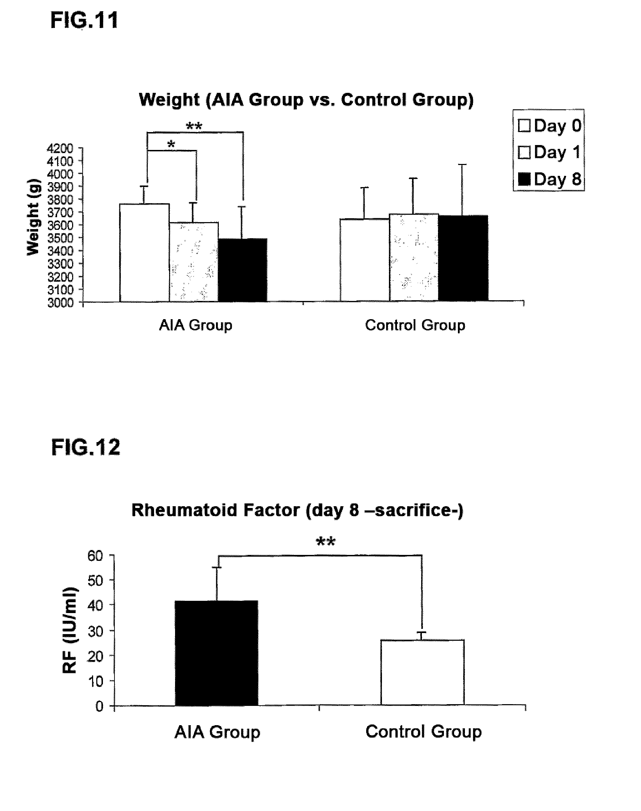

Figure 11 shows the weight of the rabbits following the induction of AIA. AIA causes a weight loss in the group of 13 New Zealand white rabbits (AIA Group) wherein the arthritis was induced. * p < 0.05; ** p < 0.01. In the group of 6 New Zealand white rabbits wherein AIA was not induced (Control Group), no weight reduction is observed at the time of sacrifice. The results obtained from the measurement of the weight are shown as the mean ± the standard deviation of the animals in each group.
Figure 12 shows the analysis of the expression of the rheumatoid factor following the induction of AIA. AIA produces an increase in the rheumatoid factor (RF) in the AIA group of New Zealand white rabbits wherein the arthritis was induced as compared to the Control group. ** p < 0.01. The results obtained from the measurement of the RF are shown as the mean ± the standard deviation of the animals in each group.

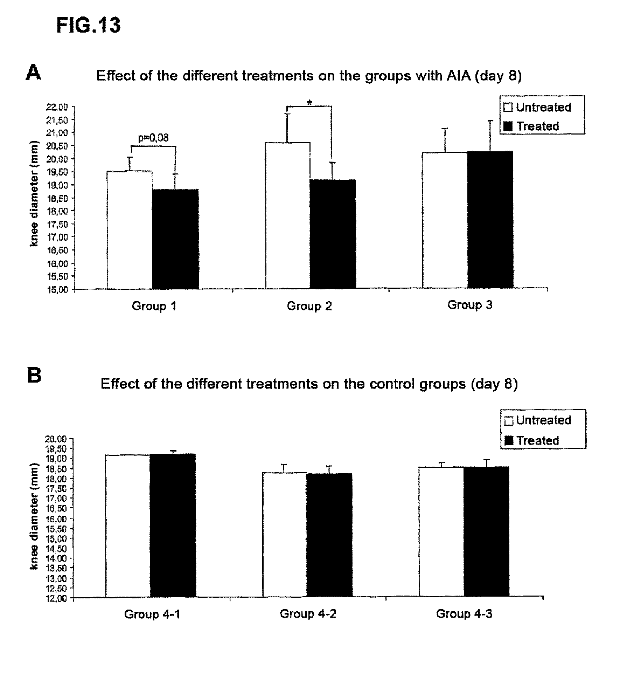

Figure 13 shows the effect of the treatments (5 µg APO2L / TRAIL) on AIA in the different groups of New Zealand white rabbits. On the day of the sacrifice, the latero-lateral diameter of all the knees of all the rabbits is measured. A. The bars indicate the mean ± the standard deviation of the untreated knees (white bars) and the treated knees (black bars) in group 1 (APO2L / TRAIL), group 2 (LUVs-APO2L / TRAIL) and group 3 (LUVs). * p < 0.05. B. The bars indicate the mean ± the standard deviation of the untreated knees (white bars) and the treated knees (black bars) in control group 4-1 (APO2L / TRAIL), group 4-2 (LUVs-APO2L / TRAIL) and group 4-3 (LUVs).

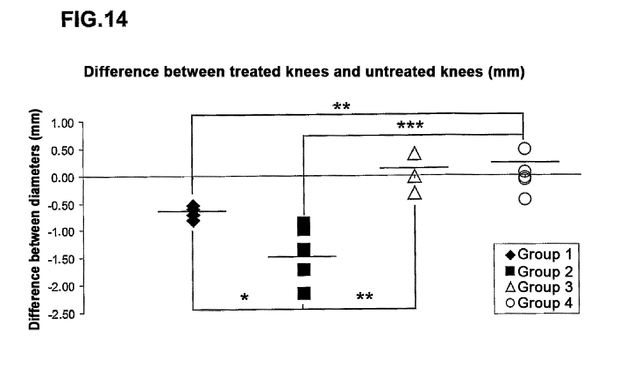

Figure 14 shows the effect of the treatments on AIA in each New Zealand white rabbit. In each rabbit, the difference between the latero-lateral diameter of the treated knee and the untreated knee is calculated and expressed in millimetres (mm). Rabbits in group 1 (black diamonds, APO2L /TRAIL), group 2 (black squares, LUVs-APO2L / TRAIL), group 3 (black triangles, LUVs) and group 4 (white circles, control). * p < 0.05; ** p < 0.01; *** p < 0.001.

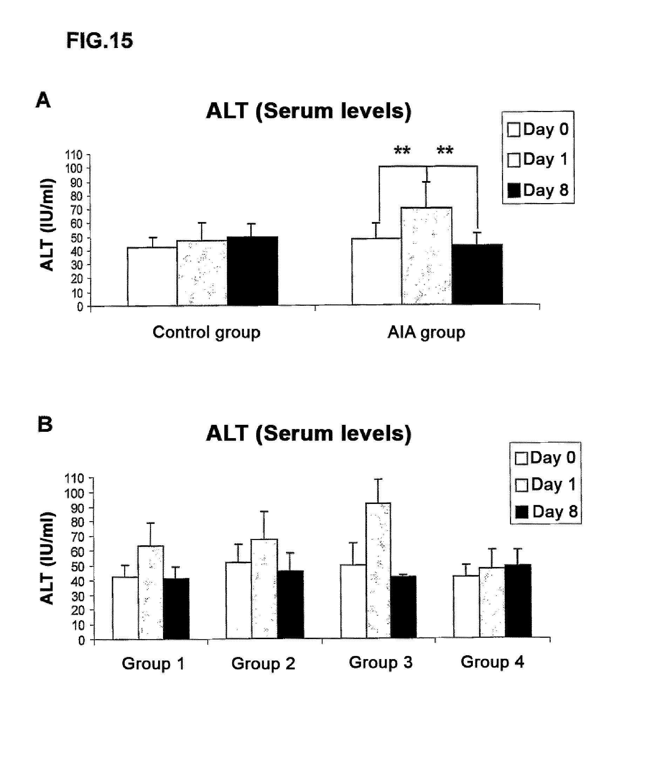

Figure 15 shows the absence of systemic hepatotoxic effect of the treatments in the different groups of New Zealand white rabbits. A. Following the induction of AIA, there is a transitory increase in the levels of alanine aminotransferase (ALT) in the serum of the rabbits in the AIA group, caused by the induction of arthritis and not the histidine tail of APO2L / TRAIL-His₁₀. ** p < 0.01. B. This transitory increase in ALT is observed in the 3 treatment groups. In the Control group, there are no variations in the serum levels of ALT between the beginning of the experiment and the day of the sacrifice. Los results obtained from the measurement of ALT are shown as the mean ± the standard deviation of the animals in each group.

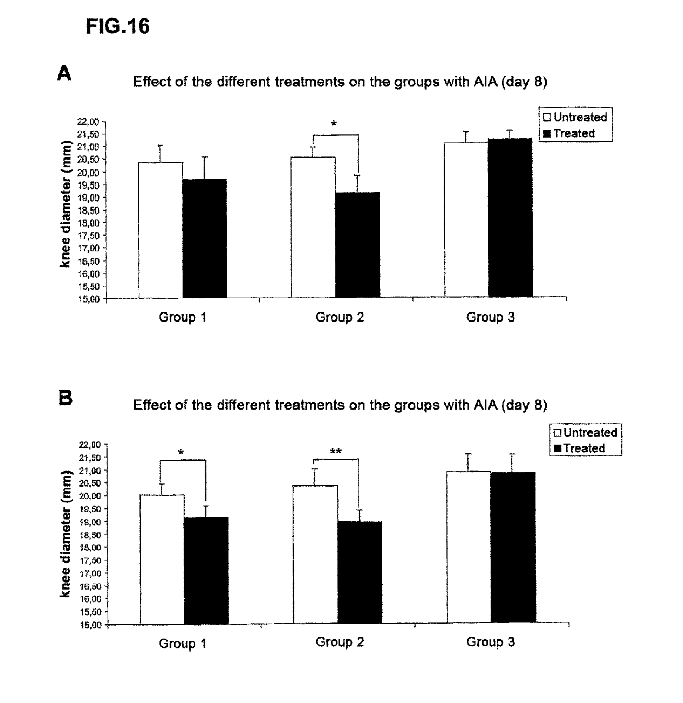

Figure 16 shows the effect of the treatments (10 µg APO2L / TRAIL) on the latero-lateral diameter of the knees in the different groups of New Zealand white rabbits. A. Experiment performed on 10 New Zealand white rabbits (Group 1, N=4; Group 2, N=4; Group 3, N=2). B. Experiment performed on 13 New Zealand white rabbits (Group 1, N=5; Group 2, N=5; Group 3, N=3). The bars indicate the mean ± the standard deviation of the untreated knees (white bars) and the treated knees (white bars) in group 1 (APO2L / TRAIL), group 2 (LUVs-APO2L / TRAIL) and group 3 (LUVs). * p < 0.05; ** p < 0.01.

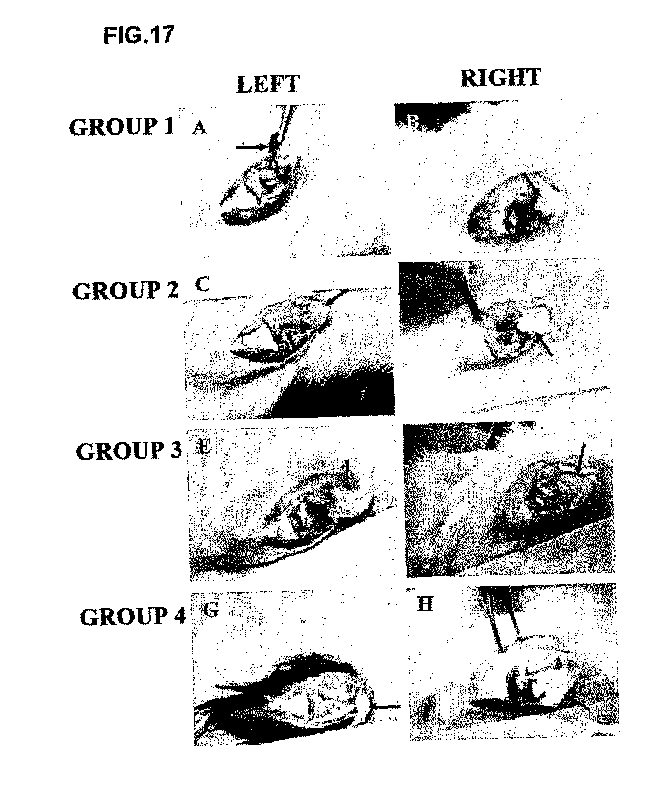

Figure 17 shows the macroscopic examination of the knees in the different groups of New Zealand white rabbits. The figure shows a representative example of a rabbit from each group. A. Left knee group 1 (not treated). B. Right knee group 1 (treated with APO2L / TRAIL). C. Left knee group 2 (not treated). D. Right knee group 2 (treated with LUVs-APO2L /TRAIL). E. Left knee group 3 (not treated). F. Right knee group 3 (treated with LUVs). G. Left knee group 4 (control, not treated). H. Right knee group 4 (control, treated with LUVs-APO2L / TRAIL). The black arrow indicates the location of the retro-patellar synovial bursa.

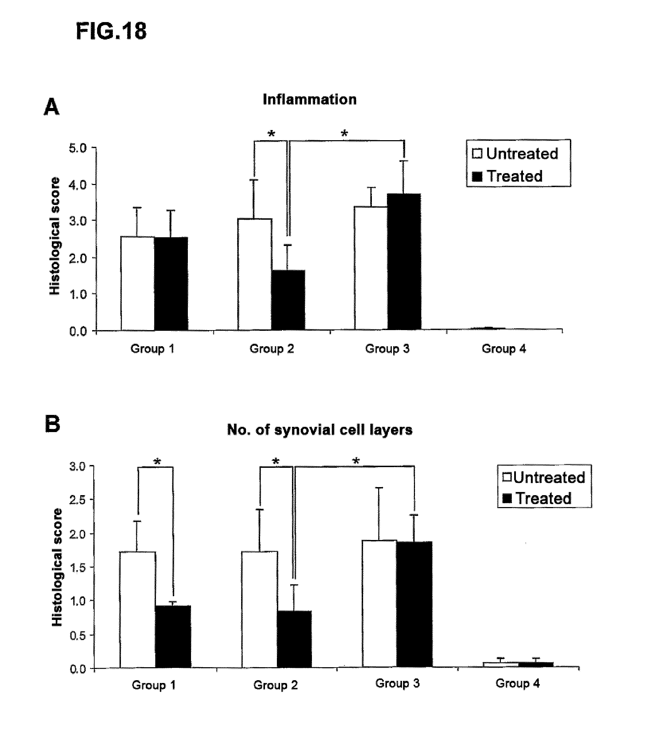

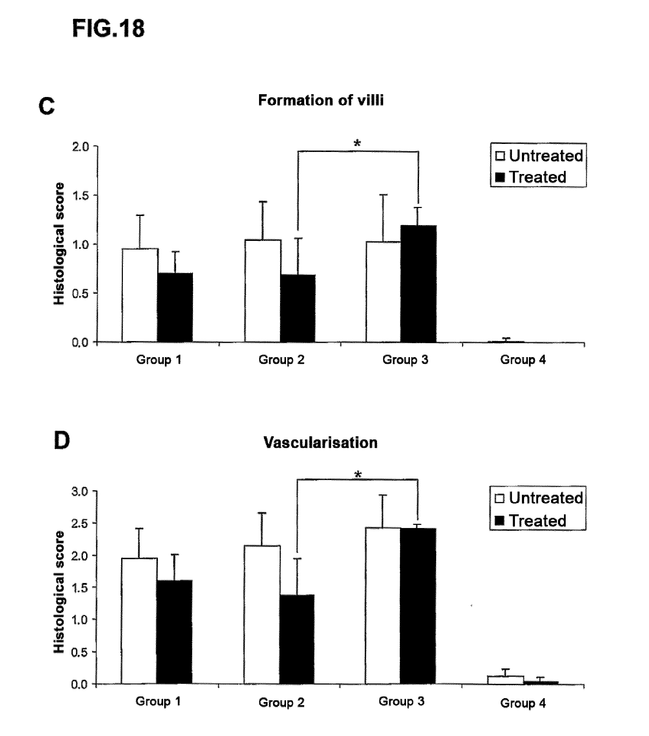

Figure 18 shows the evaluation of the histopathological parameters of the knees in the different groups of New Zealand white rabbits. Evaluation of the 4 histological parameters described by Sdnchez-Pernaute O. et al. (Sdnchez-Pernaute O. et al. Arthritis Rheum 1997; 40: 2147-56) for the evaluation of inflammation in the New Zealand white rabbit model of AIA. A. Inflammatory infiltrate. B. Synovial hyperplasia (number of layers of synovial cells). C. Formation of villi. D. Increase in vascularisation. In each slide, the entire synovial tissue present was examined under an optical microscope (20x) and the result was expressed as the mean ± the standard deviation of all the fields examined for each of the 4 parameters.

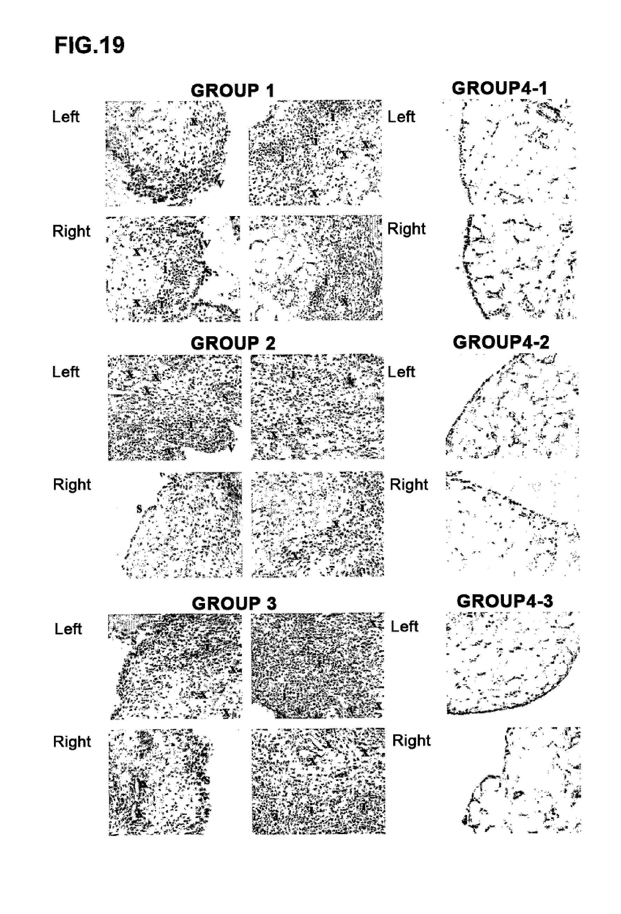

Figure 19 shows the microscopic examination of the knees in the different groups of New Zealand white rabbits. Staining with haematoxylin-eosin (H / E) of the synovial tissue of the knees of the rabbits. The figure shows a representative example of a rabbit from each group (20x). Left: Untreated left knee; Right: Treated right knee (group 1: APO2L / TRAIL; group 2: LUVs-APO2L / TRAIL; group 3: LUVs). The image indicates the presence of inflammatory infiltrate (i), the layer of synovial cells (s), the presence of villi (v) and the presence of blood vessels (x).

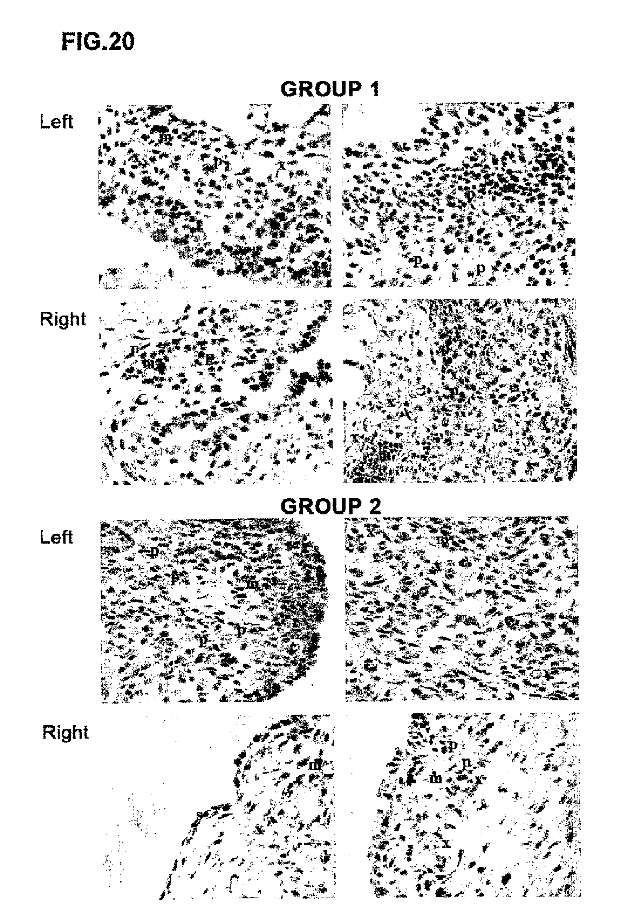

Figure 20 shows the microscopic examination of the knees in the different groups of New Zealand white rabbits. Staining with H / E of the synovial tissue of the knees of the rabbits. The figure shows a representative example of a rabbit from each group (40x). Left: Untreated left knee; Right: Treated right knee (group 1: APO2L / TRAIL; group 2: LUVs-APO2L / TRAIL). The image indicates the presence of mononuclear inflammatory infiltrate (m), polymorphonuclear inflammatory infiltrate (p), the layer of synovial cells (s) and the presence of blood vessels (x).

### EXAMPLES

The following specific examples provided in this patent document serve to illustrate the nature of the present invention. These examples are included for illustrative purposes only and should not be interpreted as limitations to the invention claimed herein. Therefore, the examples described further below illustrate the invention without limiting the field of application thereof.

### Example 1. Generation of liposomes decorated with bioactive recombinant APO2L / TRAIL

### Expression of the recombinant APO2L / TRAIL protein

In order to obtain the recombinant APO2L / TRAIL protein, prokaryotic cell expression vector *pET19b* (Novagen, Gibbstown, NJ) was used with the cDNA cloned from the soluble portion of the human APO2L / TRAIL protein (codons 114-281), which expresses a 10-histidine tail (Pitti RM. et. al. J Biol Chem 1996; 271(22): 12687-90). Plasmid *pET19b* contains an ampicillin resistance gene and its promoter responds to induction with IPTG (isopropyl-beta-D-thiogalactoside). Said plasmid was used in the E. Coli BL21-CodonPlus® (DE3)-RP strain (Stratagene, La Jolla, CA), which contains an additional plasmid with a chloramphenicol resistance gene.

In the first place, competent bacteria of said E. Coli strain were obtained using a method based on the opening of the membrane pores by means of CaCl₂ at a low temperature. After culturing the bacteria, and once it reaches the exponential growth phase, the culture is cooled in ice for 30 minutes in order to stop the growth, and the bacteria are re-suspended in a buffer containing 0.1 M CaCl₂, 0.07 M MgCl₂ and 0.04 M Sodium Acetate, keeping them in the cold for 15 minutes, such that the pores are formed in the bacterial membrane; subsequently, the bacteria are centrifuged at 3.500 rpm for 10 minutes at 4 ºC, the supernatant is eliminated, and they are re-suspended in a conservation buffer containing 0.1 M CaCl₂ and 15 % Glycerol, and kept at -80 °-C until they are to be used.

The competent E. Coli BL21-CodonPlus® (DE3)-RP bacteria are transformed with 50 ng of the *pET19b-APO2L* / *TRAIL* construct following the instructions of the commercial firm Stratagene. To this end, a method based on thermal shock was used, incubating the competent bacteria in ice and, subsequently, in a wet bath at 42 °C. The transformed bacteria are seeded in an LB-Agar plate containing 100 µg / ml Ampicillin and cultured overnight at 37 °C. On the following day, one of the transformed colonies is selected and grown in 10 ml of LB medium containing 100 mg / ml Ampicillin and 50 mg / ml Chloramphenicol, and left under culture at 37 °C, under stirring at 200 rpm for 24 hours. Once this time has elapsed, a 1 / 100 dilution of the bacterial culture in antibiotic-free LB medium is performed, and it is allowed to grow for 4-6 hours, until the transformed bacteria are in the exponential growth phase (absorbance reading at 600 nm of 0.6-0.8); at this time, the expression of recombinant APO2L / TRAIL is induced by adding 1 mM IPTG to the bacterial culture and incubating it at 37 °C under stirring for 18 hours. Subsequently, the induced bacterial culture is collected by means of centrifugation at 3,500 rpm for 30 minutes, the supernatant is eliminated and the pellet is kept at -20 °C until it is to be used.

### Folding and Purificación of the recombinant APO2L / TRAIL protein

For the purification of APO2L / TRAIL, use has been made of the fact that said protein is expressed as a fusion protein bound to a 10-histidine tail (APO2L / TRAIL-His₁₀), which shows affinity for bivalent metals such as Cobalt (Co²⁺). For this reason, the purification was performed by means of immobilised metal affinity chromatography using the BD TALON TM Metal Affinity Resin (Clontech Laboratories, Inc., Mountain View, CA), which contains Co²⁺.

In the first place, the bacterial pellet is washed with a buffer containing 0.05 M Sodium Phosphate, pH 7.4, 1 M NaCl, 6 M Urea and 0.5 % Triton X-100, centrifuged at 3,500 rpm for 30 minutes. The supernatant is discarded and the pellet is re-suspended in a solubilisation buffer containing 0.05 M Tris-HCl, pH 8.5, 5 M Guanidinium Chloride and 0.03 M DTT, and incubated at 4 °C under stirring for 3 hours. The supernatant, which contains APO2L / TRAIL-His₁₀, is collected by means of centrifugation at 20,000 g for 30 minutes at 4 °C and diluted by adding it drop by drop on folding buffer 1, which contains 0.05 M Tris-HCl, pH 7.4, 0.4 M L-Arginine, 2 mM DTT, 0.5 M Urea and 0.5 M NaCl, under stirring at 4 °C. The dilute obtained is dialised against folding buffer 2, which contains 0.05 M Tris-HCl, pH 7.4, 1 mM DTT, 0.1 M Urea, 0.3 M NaCl, overnight at 4 °C. Subsequently, the solution is centrifuged at 20,000 rpm for 30 minutes at 4 °C, to obtain the supernatant, where we find APO2L / TRAIL-His₁₀ correctly folded, and incubated with a resin containing Co²⁺ following the manufacturer's instructions for the purification of recombinant proteins under native conditions. Finally, APO2L / TRAIL-His₁₀ is eluted from the resin by means of the buffer containing 0.05 M Sodium Phosphate, pH 7.0, 0.3 M NaCl and 0.15 M Imidazole. The collected recombinant protein is unified and the Imidazole is eliminated by changing to another dilution buffer called KHE (0.1 M KCI, 10 mM Hepes, 0.1 mM EDTA, pH 7.0), using the Disposable PD-10 Desalting Column (Amersham Biosciences, Uppsala, Sweden), or concentrated using the Amicon Ultra-15 Centrifugal Filter 10,000 NMWL (Millipore, Billerica, MA), following, in both cases, the manufacturer's instructions. The protein obtained is kept at 4 °C until it is to be used.

Aliquots are saved from all the purification steps and subsequently analysed by means of 12 % polyacrylamide-SDS gel electrophoresis (SDS-PAGE) and Coomassie blue staining (Figure 1.A.). The expression of recombinant APO2L / TRAIL is induced in the culture of transformed, treated E. Coli BL21 -CodonPlusⓇ (DE3)-RP bacteria with 1 mM IPTG for 18 hours; this increases the expression of the 22-kDa molecular weight band, which corresponds to the APO2L / TRAIL-His₁₀ fusion protein (Figure 1.A.: 1 and 2). When folding the protein by means of the dilution and dialysis process, a precipitate appears which contains most of the bacterial proteins in addition to APO2L / TRAIL- His₁₀ in the form of unsolubilised inclusion bodies (Figure 1.A.: 3). In the supernatant obtained, we find the recombinant protein correctly folded (Figure 1.A: 4). After incubating the diluted, dialysed extract with the resin containing Co²⁺, the intensity of the band corresponding to APO2L / TRAIL-His₁₀ is considerably reduced, which indicates that APO2L / TRAIL-His₁₀ has remained for the most part bound to the resin (Figure 1.A: 5). Finally, after eluting the recombinant protein from the resin with 0.15 M Imidazole, the band corresponding to APO2L / TRAIL-His₁₀ appears in the elution fraction (Figure1.A: 7).

Furthermore, a Western-blot of the elution fraction was performed, by developing with the His.TagⓇ Monoclonal Antibody (Novagen, Gibbstown, NJ), which recognises the histidine tail that includes most of the expression vectors from Novagen, such as *pET19b,* the Purified Mouse Anti-Human TRAIL monoclonal antibody (BD Biosciences, Franklin Lakes, NJ) and with a rabbit polyclonal antiserum, previously generated in our laboratory, which recognises APO2L / TRAIL. Development of the Western-blot of the resin eluate using the anti-His Tag monoclonal antibody, the anti-TRAIL monoclonal antibody or a rabbit polyclonal antiserum that recognises APO2L / TRAIL confirms that the 22-kDa band observed in the staining with Coomassie blue is the recombinant APO2L / TRAIL-His₁₀ protein (Figure 1.B.).

The APO2L / TRAIL protein is a deadly ligand that induces apoptosis through the extrinsic pathway. In order to check the bioactivity of the purified protein, a cytotoxicity assay is performed on the Jurkat clone E6.1 cell line and the RPMI 8226 cell line. After 24 hours of culture in the presence of APO2L /TRAIL-His₁₀, in both cases the presence of numerous cells with the typical apoptotic morphology is observed (Figure 2).

### Generation of liposomes decorated with recombinant APO2L / TRAIL

The lipids used to generate the liposomes are the following: egg Phosphatidylcholine (PC), porcine brain Sphingomyelin (SM), bovine Cholesterol (CHOL) and 1,2-dioleoyl-sn-glycero-3-[(N-(5-amino-1-carboxypentyl)iminodiacetic acid)succinyl] functionalised with nickel (DOGS-NTA-Ni) (DOGS-NTA-Ni) (Avanti Polar Lipids Inc., Alabaster, AL). The mixture of lipids is prepared in Chloroform, dried in glass tubes by means of a Nitrogen flow for 30 minutes and, subsequently, by means of a vacuum for 2 hours. The lipids are re-suspended in KHE buffer and the suspension is subjected to 10 freezing / thawing cycles; subsequently, it is subjected to extrusion through Polycarbonate membranes with a 200-nm pore (Whatman, Maidstone, UK) 10 times, using an Extruder (Avanti Polar Lipids Inc., Alabaster, AL), in order to obtain the LUV-type (Large Unilamellar Vesicles) liposomes. The LUVs generated have a lipid composition with a mixture of PC, SM, CHOL and DOGS-NTA-Ni in a proportion of 55:30:10:5, similar to the known composition of natural exosomes that contain deadly ligands physiologically secreted by cells. Moreover, LUVs were generated with a proportion of DOGS-NTA-Ni of 0, 1, 2.5 % (w / w), maintaining the same proportion of lipids described above, except for that of PC.

The LUVs are incubated with the APO2L / TRAIL-His₁₀ protein at a protein:lipid molar ratio of 1:2,000 for 30 minutes at 37 ºC. Subsequently, the LUVs-APO2L / TRAIL are collected by means of ultracentrifugation at 60,000 rpm for 5 hours at 4 ºC, and the pellet obtained is re-suspended in sterile KHE buffer and kept at 4 ºC until it is to be used.

The binding of APO2L / TRAIL to the LUVs is analysed by means of 12 % polyacrylamide-SDS gel electrophoresis (SDS-PAGE) and Coomassie blue staining of both the pellet and the supernatant obtained (Figure 3).

The concentration of the protein bound to the LUVs is evaluated by means of the BCA and Bradford quantification methods and the lipid concentration of the LUVs is evaluated using Fiske's method.

It is verified that the binding of APO2L / TRAIL-His₁₀ to the LUVs is dependent on the presence of the lipid DOGS-NTA-Ni, such that, when the protein is incubated with LUVs with 5 % of DOGS-NTA-Ni, it binds to the liposomes and is found in the pellet (Figure 3.: 2), whereas, when APO2L /TRAIL-His₁₀ is incubated with LUVs without DOGS-NTA-Ni, all the protein appears in the supernatant (Figure 3.: 9). The incubation of the recombinant protein with the LUVs with 2.5 % and 1 % of Nickel-chelating lipid does not manage to bind the entire protein, and a part thereof appears in the supernatant and a part appears bound to the LUVs in the pellet (Figure 3.: 4 and 6; 5 and 7, respectively).

In the studies detailed below, the LUVs-APO2L / TRAIL are used with a proportion of DOGS-NTA-Ni of 5 % (w / w).

In order to check the bioactivity of the LUVs-APO2L / TRAIL, a cytotoxicity assay is performed on the human Jurkat clone E6.1 tumour cell line (Figure 4.). After 24 hours under culture, we may observe that the LUVs 5 % Ni-APO2L / TRAIL present in the pellet induce the apoptosis of most of the cells, whereas incubation of the cells with the same quantity of supernatant does not induce the death thereof. Moreover, the cytotoxicity induced by the LUVs-APO2L / TRAIL is due to the presence of the recombinant protein, since incubation in the presence of control 5 % LUVs (not incubated with APO2L /TRAIL-His₁₀) does not induce apoptosis.

### Example 2

### Use of the LUVs-APO2L / TRAIL for the treatment of a tumour or a cancer

The use of various recombinant forms of TRAIL / APO2 in *in vitro* and *in vivo* pre-clinical trials to induce the apoptosis of tumour cells, either in monotherapy, or combined with other therapies, has been disclosed. In fact, the use thereof for the treatment of various malignant neoplasms is currently being evaluated in various clinical trials (Johnstone et al. Nat Rev Cancer. 2008 Oct; 8: 782-98). The present specification evaluates the capacity of the LUV-APO2L / TRAIL liposomes to induce cell death in different tumour cell lines of human origin. The *in vitro* cytotoxicity studies performed have demonstrated that the capacity to induce cell death with LUV-APO2L / TRAIL is greater than with soluble APO2L / TRAIL. Therefore, the use of liposomes decorated with APO2L / TRAIL is an effective therapy for the treatment of cancer.

### MATERIAL AND METHODS

After obtaining and optimising the LUVs-APO2L / TRAIL, studies of their bioactivity were performed, analysing their capacity to induce cell death *in vitro* in different tumour cell lines, in parallel with soluble APO2L / TRAIL-His₁₀.

To this end, cell death induction assays were performed on different tumour cell lines: Jurkat clone E6.1 (from a human T-type acute lymphoblastic leukemia), RPMI 8226 (from a human plasmacytoma of B origin), U266 (from a human myeloma / plasmacytoma of B origin), IM-9 (from a human myeloma /plasmacytoma of B origin), MM1.S (from a human myeloma / plasmacytoma of B origin), A549 (from a human lung adenocarcinoma), U937 (from a histiocytic lymphoma) and U937-Bcl2 (from the U937 parental line wherein the antiapoptotic Bcl2 protein has been overexpressed).

Dose-response assays were performed by incubating the Jurkat clone E6.1 and RPMI 8226 lines with the following doses: 10, 25, 50, 100, 250, 500 and 1,000 ng / ml, and the rest of the lines with the doses: 10, 50, 100, 500 and 1,000 ng / ml, of both soluble APO2L / TRAIL-His₁₀ and LUVs-APO2L / TRAIL for 24 hours, and the cell death was evaluated by means of Trypan blue staining, using a variant of Mossman's MTT (dimethyl-thiazolyl-tetrazolium) reduction method, and by means of analysis by flow cytometry of the translocation of Phosphatidylserine to the outer face of the cell membrane by means of the binding of Annexin-V conjugated to Phycoerythrin (PE), in the case of the Jurkat clone E6.1, U266, IM-9, MM1.S and A549 cell lines, or with Fluorescein (FITC) in the case of the RPMI 8226 cell line.

The cytotoxicity assays were performed in the absence or the presence of the blocking anti-APO2L / TRAIL antibody called 5C2 (gently donated by Dr. Ashkenazi, Genentech, San Diego, CA), in order to verify that the cytotoxicity observed in the cell lines incubated with both soluble APO2L / TRAIL-His₁₀ and with LUVs-APO2L / TRAIL is specific and caused by this deadly ligand.

The results of the cytotoxicity studies are shown as the mean ± standard deviation of at least 2 different experiments.

For all the comparison studies between quantitative variables, Student's t-test was applied.

In all cases, a value of p < 0.05 has been considered to be statistically significant.

The statistical data were analysed using the SPSS for Windows Release 10.0 software (SPSS Inc., Chicago, lL).

### RESULTS

In the first place, the *in vitro* cytotoxicity was analysed on different tumour cell lines of human origin, generating parallel dose-response curves for soluble APO2L / TRAIL and LUVs-APO2L / TRAIL, and analysing the results by means of Trypan blue staining, using a variant of Mossman's method or by means of analysis by flow cytometry of the positive Annexin-V cells (apoptotic cells).

The results of the cytotoxicity assays with the Jurkat clone E6.1 line show that the LUVs-APO2L / TRAIL have a greater cytotoxic effect than soluble APO2L / TRAIL. When the capacity to induce cell death is analysed in parallel using any of the aforementioned methods, it is observed that the mean inhibitory concentration (lC50) is clearly lower (between 3 and 7 times) in the case of the liposomes decorated with APO2L / TRAIL as compared to soluble APO2L / TRAIL (Figure 5.).

In the case of the RPMI 8226 cell line, the LUVs-APO2L / TRAIL also induce cell death more efficiently than soluble APO2L / TRAIL, and an lC50 approximately 2 times lower is observed in the case of the LUVs-APO2L /TRAIL as compared to soluble APO2L / TRAIL (Figure 6.).

In order to verify that the cytotoxic effect observed in these experiments is caused by APO2L / TRAIL, the cytotoxicity assays are performed in the presence of the blocking anti-APO2L / TRAIL 5C2 antibody. In both tumour lines, the cytotoxic effect of APO2L / TRAIL is reverted when the incubation is performed in the presence of 5C2. In the case of soluble APO2L / TRAIL, a dose of 100 ng / ml of 5C2 is necessary to achieve inhibition of the cell death produced by APO2L / TRAIL at a dose of 100 ng / ml, whereas, in the case of the LUVs-APO2L / TRAIL, in order to inhibit the cell death produced by the same 100-ng / ml dose, it is necessary to use a 1,000-ng / ml dose of 5C2, which indicates that the efficiency of the apoptotic signal is greater in the case of the LUVs-APO2L / TRAIL (Figure 7.).

Finally, the cytotoxicity study was expanded to other tumour cell lines of human origin: U266, IM-9, MM1.S, A549, U937 and U937-Bcl2 (Figure 8.). The analyses indicate that, in all of them, the capacity to induce cell death is greater in the case of the LUVs-APO2L / TRAIL than in the case of soluble APO2L /TRAIL when analysed in parallel. Even in the U937-Bcl2 line, the LUVs-APO2L / TRAIL are capable of overcoming the antiapoptotic effect of the Bcl2 protein more effectively than soluble APO2L / TRAIL.

### Example 3

### Use of the LUVs-APO2L / TRAIL for the treatment of RA

On the other hand, APO2L / TRAIL is also involved in the regulation of the immune system and in the pathogenesis of inflammatory and autoimmune diseases, such as rheumatoid arthritis (RA). The use of recombinant APO2L /TRAIL for the treatment of RA has been disclosed (Yao et al. 2006. Arthritis Res Ther 8: R16). In the present specification, an animal model of antigen-induced arthritis (AIA) is used to demonstrate that the intra-articular injection of LUV-APO2L / TRAIL is capable of reducing the inflammation of the joints affected by arthritis more effectively than when the soluble APO2L / TRAIL protein is injected. Therefore, the use of liposomes decorated with APO2L / TRAIL is an effective therapy for the treatment of inflammatory or autoimmune diseases, such as RA.

### MATERIAL AND METHODS

### Induction of antigen-induced arthritis (AIA) in New Zealand white rabbits

On the basis of the prior scientific literature (Sanchez-Pernaute O. et al. Rheumatol 2003; 42: 19-25; Lo YJ. Et al. Rheumatol Int 2007; 27(12): 1099-111; Keravala A. et al. Arthritis Res Ther 2006; 8(4): R91; Nakaya H. et al. Arthritis Rheum 2005; 52: 2559-2566; Yao Q. et al. Artritis Res Ther 2006; 8(1): R16), AIA was induced in New Zealand white rabbits in order to evaluate the efficacy of the treatment with LUVs-APO2L / TRAIL *in vivo* in an animal model of arthritis. To this end, New Zealand white rabbits were sensitised by means of two intradermal (i.d.) injections of 5 mg of ovalbumin (OVA, Sigma, St. Louis, MO) with an authorised adjuvant.

Subsequently, AIA was induced in both by means of the intra-articular (i.a.) injection of 5 mg of OVA dissolved in sterile PBS. The protocol used for the induction of AIA is schematically represented in Figure 9.

### Induction of antigen-induced arthritis (AIA) in New Zealand white rabbits

On the basis of the prior scientific literature (Sanchez-Pernaute O. et al. Rheumatol 2003; 42: 19-25; Lo YJ. Et al. Rheumatol Int 2007; 27(12): 1099-111; Keravala A. et al. Arthritis Res Ther 2006; 8(4): R91; Nakaya H. et al. Arthritis Rheum 2005; 52: 2559-2566; Yao Q. et al. Artritis Res Ther 2006; 8(1): R16), AIA was induced in New Zealand white rabbits in order to evaluate the efficacy of the treatment with LUVs-APO2L / TRAIL *in vivo* in an animal model of arthritis. To this end, New Zealand white rabbits were sensitised by means of two intradermal (i.d.) injections of 5 mg of ovalbumin (OVA, Sigma, St. Louis, MO) with an authorised adjuvant.

### Use of the LUVs-APO2L / TRAIL for the treatment of AIA

The experiment was performed on 19 New Zealand white rabbits distributed into the following groups on the basis of the treatment received:
1. APO2L group: 5 rabbits which, 24 hours after having AIA induced in both knees, had their right knee treated by means of an i.a. injection of 5 µg of soluble APO2L / TRAIL-His₁₀ in 0.5 ml of sterile PBS, whereas 0.5 ml of sterile PBS was injected in their left knee.
2. LUVs-APO2L group: 5 rabbits which, 24 hours after having AIA induced in both knees, had their right knee treated by means of an i.a. injection of LUVs decorated with 5 µg of APO2L / TRAIL in 0.5 ml of sterile PBS, whereas 0.5 ml of sterile PBS was injected in their left knee.
3. LUVs group: 3 rabbits which had AIA induced in both knees and, 24 hours later, their right knee was treated by means of an i.a. injection of LUVs in 0.5 ml of sterile PBS at the same concentration as in group 2, whereas 0.5 ml of sterile PBS was injected in their left knee.
4. Control Group: 6 rabbits which received the 2 sensitisation injections and wherein the i.a. injection in both knees was performed with 0.5 ml of PBS. In these rabbits, no treatment was performed on the left knee, whereas the following treatments were performed on the right knee, depending on the subgroup:
   4.1. Control-APO2L: 2 rabbits were injected with soluble APO2L / TRAIL-His₁₀ in 0.5 ml of sterile PBS.
   4.2. Control-LUVs-APO2L: 2 rabbits were injected with liposomes decorated with APO2L / TRAIL in 0.5 ml of sterile PBS.
   4.3. Control-LUVs: 2 rabbits were injected with LUVs in 0.5 ml of sterile PBS.

On the one hand, this last group serves as a healthy control (untreated knees) and, on the other hand, as a negative control to verify the lack of effect of the treatments in the absence of AIA (treated knees).

The follow-up of the rabbits was performed for 8 days from the AIA-induction i.a. injection (7 days from the treatments received, depending on the group whereto they belong) and was based on the daily measurement of the latero-lateral diameter of the knees and the measurement of the weight of each animal. After these 8 days, the animals were sacrificed, a macroscopic examination of the open knees was performed and the retro-patellar synovial bursa of both knees was subjected to surgical exeresis. The tissues were fixed in 10 % formaldehyde and, subsequently, they were paraffined and cut in a microtome in serial slides 5 microns thick, for subsequent histological study by staining with Haematoxylin / Eosin (H / E, Merck, Darmstadt, Germany).

Moreover, blood was extracted from all the animals on days 31 (day of the induction of AIA prior to the i.a. injection), 32 (day of the treatments prior to the i.a. injection) and 39 (day of the sacrifice), in order to analytically determine the rheumatoid factor (RF) and the alanine aminotransferase (ALT) transaminase.
2 additional experiments were performed with 10 and 13 rabbits, respectively, following the same protocol described above, using a 10-µg dose of APO2L / TRAIL.

### Histopathological evaluation of AIA

The histopathological evaluation of the degree of synovial inflammation present in the cuts stained with H / E was performed on the basis of the scale previously described for this animal model of arthritis (Sanchez-Pernaute O. et al. Arthritis Rheum 1997; 40: 2147-56, Table 1).

The following criteria were analysed: presence of inflammatory infiltrate, increase in the number of layers of synovial cells, formation of villi and increase in vascularisation, with a score ranging between 0, for healthy tissue, and 13, for the most severe inflammation. All the synovial tissue present in each slide was examined by optical microscopy at a magnification of 20x without knowing the group whereto said slide belonged, and the result was expressed as the mean ± the standard deviation of all the fields examined.

**TABLE 1. Criteria for the histological evaluation of AIA**

| Parameter | Score |
|---|---|
| Inflammation | 0 = Normal |
| | 1 = Minimal inflammatory infiltrate |
| | 2 = Medium inflammatory infiltrate |
| | 3 = Moderate inflammatory infiltrate |
| | 4 = Marked inflammatory infiltrate |
| | 5 = Severe inflammatory infiltrate |
| No. of layers of synovial cells | 0 = Normal (1-2 layers of cells) |
| | 1 = Mild hyperplasia (2-3 layers of cells) |
| | 2 = Moderate hyperplasia (3-5 layers of cells) |
| | 3 = Marked hyperplasia (5 or more layers of cells) |
| Formation of villi | 0 = Normal |
| | 1 = Minimal (1-2 villi) |
| | 2 = Severe (3 or more villi) |
| Vascularisation | 0 = Normal (limited number of vessels) |
| | 1 = Mild hypervascularisation (focal presence of scant small vessels) |
| | 2 = Moderate hypervascularisation (focal presence of numerous small vessels) |
| | 3 = Marked hypervascularisation (numerous widely-distributed vessels) |

Based on: Sanchez-Pernaute 0, et al. Arthritis & Rheum 1997; 40: 2147-56

### Statistical analysis

The results obtained from the measurement of the latero-lateral diameter of the knees are shown as the mean ± the standard deviation of the measurements of the means of the animals in each group independently taken by two observers. The results obtained from the measurement of the weight loss, the RF and the ALT are shown as the mean ± the standard deviation of the animals in each group.

The results of the histopathological analysis of the cuts stained with H / E are shown as the mean ± the standard deviation of all the fields examined in each slide.

For all the comparison studies between quantitative variables, Student's t-test was applied.

In all cases, a value of p < 0.05 was considered to be statistically significant.

The statistical data were analysed using the SPSS for Windows Release 10.0 software (SPSS Inc., Chicago, lL).

### RESULTS

### Induction of antigen-induced arthritis (AIA) in New Zealand white rabbits

As indicated in the section on Material and Methods, AIA is induced in both knees by means of an i.a. injection of 5 mg of OVA in 13 previously sensitised New Zealand white rabbits (AIA Group). In 6 sensitised rabbits, the i.a. injection is performed with PBS (Control Group).

Twenty-four hours after the induction, the arthritis is correctly generated in all the knees of all the rabbits in the AIA group, and there is a statistically significant increase in the latero-lateral diameter of all the knees, whereas in the Control group, the i.a. injection of PBS does not produce a significant increase in the latero-lateral diameter of the knees (Figure 10.A.). In the AIA group, the arthritis remains until the day of the sacrifice, without significant differences being observed in the measurement of the latero-lateral diameter of the knees with respect to the day of induction of AIA.

The arthritis is induced in a similar manner in both knees, without statistically significant differences being observed in the latero-lateral diameter between the left and right knees or between the 3 groups whereon the different treatments of AIA are to be performed (Group 1: APO2L; Group 2: LUVs-APO2L; Group 3: LUVs, Figure 10.B.).

As regards the weight of the animals, in the AIA group there is a statistically significant weight reduction on the day of the sacrifice with respect to the weight on the day of induction of AIA, whereas in the Control group, there is no variation in the weight throughout the experiment and until the day of the sacrifice (Figure 11.).

As regards the RF, an analytical parameter characteristic of RA, it significantly increases in the AIA group on the day of the sacrifice with respect to the values observed in the Control group (Figure 12.).

### Effect of the treatment of AIA with LUVs-APO2L / TRAIL

After verifying that the arthritis has been correctly induced, 24 hours later the treatment is performed by means of an i.a. injection. The 13 rabbits wherein AIA has been induced are randomly distributed into 3 groups: 5 rabbits whose right knee is injected with 5 µg of APO2L / TRAIL-His₁₀ by i.a. route (Group 1), 5 rabbits whose right knee is injected with 5 µg of LUVs-APO2L / TRAIL by i.a. route (Group 2) and 3 rabbits which are injected with the same concentration of LUVs as in group 2 by i.a. route, in this case without APO2L / TRAIL (Group 3). The left knees of all the rabbits in the 3 groups are injected with sterile PBS by i.a. route. These knees serve as an internal control in each animal for the natural evolution of AIA without treatment.

In the untreated knees of group 1 and group 2, the inflammation observed 24 hours after the induction remains until the day of the sacrifice. However, in the treated knees, in both group 1 (treatment with 5 µg of recombinant soluble APO2L / TRAIL) and group 2 (treatment with LUVs decorated with 5 µg of APO2L / TRAIL), a decrease in the inflammation is observed on the day of the sacrifice with respect to that observed 24 hours after the induction. When comparing the latero-lateral diameter of the untreated knees and the treated knees in each group, the decrease observed in the knees treated with LUVs-APO2L / TRAIL of group 2 is statistically significant, whereas in group 1 the decrease observed in the knees treated with soluble APO2L /TRAIL does not reach significant levels. In group 3, the i.a. injection of LUVs without APO2L / TRAIL has no effect on the inflammation, such that the treated knees present an inflammation similar to that of the untreated knees (Figure13.A.).

In the Control group, none of the 3 treatments injected in the 3 subgroups produce significant changes in the diameter of the treated knees with respect to the untreated knees; i.e. none of the 3 treatments have an effect in the absence of AIA (Figure 13.B.).

When comparing the means of the differences between the diameters of the untreated knees and the treated knees in each group, it is observed that this difference is statistically greater in group 2 than in group 1. In group 3, there are hardly any differences between the diameter of the treated knees and the untreated knees at the time of sacrifice, i.e. there was no decrease in the inflammation of the treated knees (Figure 14.).

It has been disclosed that APO2L / TRAIL-His₁₀ may have a hepatotoxic effect attributable to the presence of the histidine tail (Lawrence D. et al. Nature Med 2001; 7(4): 83-5). In order to verify that the i.a. injection of APO2L / TRAIL-His₁₀ and LUVs-APO2L / TRAIL does not present systemic toxicity, the ALT hepatic enzyme is determined in the serum of the rabbits. In the AIA group, 24 hours after the induction of arthritis, there is an increase in the serum levels of ALT and, on the day of the sacrifice, the serum levels return to values similar to the values prior to the induction of AIA (Figure 15.A.). This transitory increase and subsequent decrease to normal values on the day of the sacrifice is also observed in the 3 treatment groups separately (Figure 15.B.). In the Control group, the serum levels of ALT do not change throughout the experiment. These data indicate that the transitory increase in ALT in the rabbits with AIA is caused by the induction of arthritis and is not related to an adverse systemic effect of APO2L / TRAIL-His₁₀.

In the 2 experiments performed using a 10-µg dose of soluble APO2L /TRAIL or APO2L / TRAIL associated with LUVs, the results of the measurement of the latero-lateral diameter of the knees are similar to those of the first experiment, wherein 5 µg of APO2L / TRAIL were used (Figure 16.).

Following sacrifice of the animals, the open knees are subjected to a macroscopic examination (Figure 17.).

In the untreated left knees of the 3 treatment groups, an increase is observed in the size and the pigmentation of the retro-patellar synovial bursa, due to the inflammation produced by arthritis (Figure 17. A. C. E.). In the treated right knees of group 1 (Figure 17. B.) and, especially, in group 2 (Figure 17. D.), a lower inflammation is observed with respect to the untreated knees. In the treated knees of group 3 (Figure 17.F.), the inflammation of the retro-patellar bursa is similar to that of the untreated knees. Finally, in group 4 (Control), both the untreated and the treated knees present a normal macroscopic anatomy (Figure 17. G. H.).

The histopathological examination of the synovial tissue in the cuts stained with H / E was performed on the basis of the evaluation scale previously described by Sanchez-Pernaute O. et al. for this animal model of arthritis. As described above in the section on Material and Methods, all the synovial tissue present in each slide was examined by means of optical microscopy at a magnification of 20x and the result was expressed as the mean ± the standard deviation of all the fields examined for each of the 4 parameters analysed (Table 2).

**TABLE 2. Histopathological evaluation of AIA in the different groups**

| Knees | Group 1 | Group 2 | Group 3 | Group 4 |
|---|---|---|---|---|
| Untreated | 7.199 ± 1.309 | 7.946 ± 2.046 | 8.694 ± 1.522 | 0.139 ± 0.063 |
| Treated | 5.722 ± 1.175 | 5.159 ± 0.585 | 9.167 ± 1.449 | 0.067 ± 0.033 |

| | | | | |
|---|---|---|---|---|
| * p < 0.05 | | | | |

When the histological index is analysed by groups, the untreated knees of groups 1, 2 and 3 present high indices (approximately between 7 and 8.5, depending on the group), without statistically significant differences being observed between the groups. In the treated knees, in both group 1 and group 2, a lower histological index is observed than in the case of the untreated knees, such that, in group 2, the difference between the index of the treated and the untreated knees is statistically significant. In group 3, the histological index of the treated knees is high and similar to that of the untreated knees. The Control group presents histological indices of practically 0 (normality), and no differences are observed between the untreated knees and the knees treated with any of the 3 treatments (depending on the subgroup).

When the 4 histological parameters (inflammatory infiltrate, synovial hyperplasia, formation of villi and increase in vascularisation, Figure 18) are analysed separately, it is observed that, in group 2, the treatment with LUVs-APO2L / TRAIL is capable of reducing all of them, with a significant decrease observed in the inflammatory infiltrate and the synovial hyperplasia. In group 1, the treatment with soluble APO2L / TRAIL is capable of reducing the synovial hyperplasia, with a significant decrease being observed, the formation of villi and hypervascularisation, but it has no effect on the inflammatory infiltrate. In group 3, the treatment with LUVs has no effect on any of the 4 parameters analysed. Finally, in group 4, the index of both the untreated and the treated knees is similar and practically 0 in the 4 parameters, without any differences being observed between the different subgroups on the basis of the treatment received.

Figure 19 shows the H / E staining of the cuts of synovial tissue of the left knees (not treated) and the right knees (treated) of a representative rabbit in each group, with a magnification of 20x. In the left knees of the rabbits in group 1 (histological index: 9.381) and group 2 (histological index: 10.511), we observe an abundant inflammatory infiltrate, a marked synovial hyperplasia, with an increase in the number of layers of synovial cells (more than 5 layers of synovial cells in both), a significant increase in the number of blood vessels and the formation of villi. In the knee of the rabbit in group 1 treated with APO2L /TRAIL (histological index: 7.700), we may observe a reduction in the number of layers of synovial cells, as well as in the number of blood vessels present in the tissue as compared to the untreated knee of the same rabbit; however, we observe an abundant inflammatory infiltrate similar to that of the untreated knee. In the case of the rabbit in group 2, in the knee treated with LUVs-APO2L /TRAIL (histological index: 5.998), in addition to a significant reduction in the number of blood vessels and the synovial hyperplasia, which for the most part presents a single layer of synovial cells, we observe a significant decrease in the inflammatory infiltrate as compared to the untreated knee of the same rabbit.

In group 3, both the untreated knee (histological index: 9.833) and the knee treated with LUVs (histological index: 10.500) present an abundant inflammatory infiltrate, a marked hyperplasia of the synovial cells, a significant increase in tissue vascularisation, as well as the formation of villi, without significant differences being observed between both knees. Finally, in group 4 (Control), the synovial tissue is normal in both the untreated knees and the knees treated with any of the 3 treatments, without any differences being observed between the 3 subgroups (group 4-1: histological indices: 0.200 and 0.284; group 4-2: histological indices: 0.083 and 0.222; group 4-3: histological indices: 0.180 and 0.111).

Figure 20 shows the H / E staining of the synovial tissue of the left knee and the right knee of the same rabbits in group 1 and group 2 as in the preceding figure, in greater detail (magnification of 40x).

In the left knees of the rabbits in groups 1 and 2, we observe an abundant inflammatory infiltrate at the expense of both the mononuclear-type and the polymorphonuclear infiltrate, a marked synovial hyperplasia, with an increase in the number of layers of synovial cells (more than 5 layers of synovial cells in both), and a significant increase in the number of blood vessels. In the treated right knee of the rabbit in group 1 (APO2L / TRAIL), we may observe a decrease in the number of layers of synovial cells, as well as in the number of blood vessels present in the tissue, as compared to the untreated knee of the same rabbit; however, it may be observed that both the mononuclear and the polymorphonuclear inflammatory infiltrate persists to a similar extent as that of the untreated knee. In the treated right knee (LUVs-APO2L / TRAIL) of the rabbit in group 2, we observe a significant reduction in the number of blood vessels and in the synovial hyperplasia, which for the most part presents a single layer of synovial cells, and, also, a significant decrease in the mononuclear and the polymorphonuclear inflammatory infiltrate as compared to the untreated knee.

## Claims

1. Compound that comprises a liposome covered, at least, with the protein with amino acid sequence SEQ ID: 2 or any of the biologically active variants or portions thereof.

2. Compound according to claim 1, wherein the liposome is covered with a biologically active portion of the protein that presents an identity with SEQ ID NO: 1 of, at least, 80 %.

3. Compound according to claim 2, wherein the biologically active portion of the protein presents an identity with SEQ ID NO: 1 of, at least, 90 %.

4. Compound according to claim 3, wherein the biologically active portion of the protein presents an identity with SEQ ID NO: 1 of, at least, 95 %.

5. Compound according to claim 4, wherein the biologically active portion of the protein is SEQ ID NO: 1.

6. Compound according to any one of claims 1 to 5, wherein the protein with amino acid sequence SEQ ID: 2 or any of the variants or portions thereof has, at least, 6 histidines bound to its amino-terminal end.

7. Compound according to claim 6, wherein the protein with amino acid sequence SEQ ID: 2 or any of the variants or portions thereof has, at least, 10 histidines bound to its amino-terminal end.

8. Compound according to any one of claims 1 to 7, wherein the liposome is a large unilamellar liposome (LUV), with a diameter of between 40 nm and 500 nm.

9. Compoundd according to claim 8, wherein the LUV has a diameter of between 100 nm and 200 nm.

10. Compound according to any one of claims 1 to 9, wherein the liposome comprises at least one phospholipid selected from: phosphatidylcholine and / or sphingomyelin.

11. Compound according to claim 10, wherein the liposome further comprises a sterol.

12. Compound according to claim 11, wherein the sterol is cholesterol.

13. Compound according to any one of claims 10 to 12, wherein the liposome further comprises a chelating lipid.

14. Compound according to claim 13, wherein the chelating lipid is DOGS-NTA-Ni.

15. Use of the compound according to any one of claims 1 to 14, for the preparation of a medicament.

16. Use of the compound according to claim 15, for the preparation of a medicament designed for the prevention or treatment of a cancer.

17. Use of the compound according to claim 16, wherein the cancer is selected from: lymphoma, leukemia, plasmacytoma, myeloma or lung cancer.

18. Use of the compound according to claim 15, for the preparation of a medicament designed for the prevention or treatment of inflammatory or autoimmune diseases.

19. Use of the compound according to claim 18, wherein the inflammatory or autoimmune disease is rheumatoid arthritis.

20. Pharmaceutical composition that comprises a compound according to any one of claims 1 to 14.

21. Pharmaceutical composition according to claim 20, which further comprises a pharmaceutically acceptable vehicle.

22. Pharmaceutical composition according to any one of claims 20 or 21, which further comprises another active principle.
